# EUROPEAN PATENT SPECIFICATION

(11) **EP 1 899 325 B1**
(45) Date of publication and mention of the grant of the patent: **16.11.2011**
(21) Application number: 06763797.5
(22) Date of filing: 20.06.2006
(51) Int. Cl.: C07D 401/14, C07D 413/14, A61K 31/4166, A61P 35/00

(54) **IMIDAZOLINONE AND HYDANTOINE DERIVATIVES AS NOVEL INHIBITORS OF HISTONE DEACETYLASE**
IMIDAZOLINON- UND HYDANTOINDERIVATE ALS NEUE INHIBITOREN VON HISTONDEACETYLASE
DÉRIVÉS D'IMIDAZOLINONE ET D'HYDANTOÏNE EN TANT QUE NOUVEAUX INHIBITEURS DE L HISTONE DÉSACÉTYLASE

(30) Priority: 23.06.2005 EP 05105592
(43) Date of publication of application: 19.03.2008
(73) Proprietor: Janssen Pharmaceutica NV, 2340 Beerse (BE)
(72) Inventor: TEN HOLTE, Peter Janssen Pharmaceutica N.V., B-2340 Beerse (BE); VAN EMELEN, Kristof Janssen Pharmaceutica N.V., B-2340 Beerse (BE); ANGIBAUD, Patrick René Janssen-Cilag, F-27106 Val de Reuil Cedex (FR); MARCONNET-DECRANE, Laurence F. B. Janssen-Cilag, 27106 Val de Reuil Cedex (FR); MEERPOEL, Lieven, B-2340 Beerse (BE)
(86) International application number: PCT/EP2006/063351
(87) International publication number: WO 2006/136553

(56) References cited:
- WO-A-03/092686
- US-A1- 2005 107 384

## Description

This invention concerns compounds having histone deacetylase (HDAC) inhibiting enzymatic activity. It further relates to processes for their preparation, to compositions comprising them, as well as their use, both *in vitro* and *in vivo,* to inhibit HDAC and as a medicine, for instance as a medicine to inhibit proliferative conditions, such as cancer and psoriasis.

Nuclear histones are known as integral and dynamic components of the machinery responsible for regulating gene transcription and other DNA-templated processes such as replication, repair, recombination, and chromosome segregation. They are the subject of post-translational modifications including acetylation, phosphorylation, methylation, ubiquitination, and ADP-ribosylation,

Histone deacetylase(s), herein referred to as "HDACs", are enzymes that catalyze the removal of the acetyl modification on lysine residues of proteins, including the core nucleosomal histones H2A, H2B, H3 and H4. Together with histone acetyltransferase(s), herein referred to as "HATs", HDACs regulate the level of acetylation of the histones. The balance of acetylation of nucleosomal histones plays an important role in transcription of many genes. Hypoacetylation of histones is associated with condensed chromatin structure resulting in the repression of gene transcription, whereas acetylated histones are associated with a more open chromatin structure and activation of transcription.

Eleven structurally related HDACs have been described and fall into two classes. Class I HDACs consist of HDAC 1, 2, 3, 8 and 11 whereas class II HDACs consist of HDAC 4, 5, 6, 7, 9 and 10. Members of a third class of HDACs are structurally unrelated to the class I and class II HDACs. Class I/II HDACs operate by zinc-dependent mechanisms, whereas class III HDACs are NAD-dependent.

In addition to histones, other proteins have also been the substrate for acetylation, in particular transcriptionfactors such as p53, GATA-1 and E2F; nuclear receptors such as the glucocorticoid receptor, the thyroid receptors, the estrogen receptors; and cell-cycle regulating proteins such as pRb. Acetylation of proteins has been linked with protein stabilization, such as p53 stabilization, recruitment of cofactors and increased DNA binding. p53 is a tumour suppressor that can induce cell cycle arrest or apoptosis in response to a variety of stress signals, such as DNA damage. The main target for p53-induced cell cycle arrest seems to be the p21 gene. Next to its activation by p53, p21 has been identified by virtue of its association with cyclin/cyclin-dependent kinase complexes resulting in cell cycle arrest at both G1 and G2 phases, its up-regulation during senescence, and its interaction with the proliferating cell nuclear antigen.

The study of inhibitors of HDACs indicates that they play an important role in cell cycle arrest, cellular differentiation, apoptosis and reversal of transformed phenotypes.

The inhibitor Trichostatin A (TSA), for example, causes cell cycle arrest at both G1 and G2 phases, reverts the transformed phenotype of different cell lines, and induces differentiation of Friend leukemia cells and others. TSA (and suberoylanilide hydroxamic acid SAHA) have been reported to inhibit cell growth, induce terminal differentiation, and prevent the formation of tumours in mice (Finnin et al., Nature, 401: 188-193, 1999).

Trichostatin A has also been reported to be useful in the treatment of fibrosis, e.g. liver fibrosis and liver chirrhosis. (Geerts et al., European Patent Application EP 0 827 742, published 11 March, 1998).

The pharmacophore for HDAC inhibitors consists of a metal-binding domain, which interacts with the zinc-containing active site of HDACs, a linker domain, and a surface recognition domain or capping region, which interacts with residues on the rim of the active site.

Inhibitors of HDACs have also been reported to induce p21 gene expression. The transcriptional activation of the p21 gene by these inhibitors is promoted by chromatin remodelling, following acetylation of histones H3 and H4 in the p21 promotor region. This activation of p21 occurs in a p53-independent fashion and thus HDAC inhibitors are operative in cells with mutated p53 genes, a hallmark of numerous tumours.

In addition HDAC inhibitors can have indirect activities such as augmentation of the host immune respons and inhibition of tumor angiogenesis and thus can suppress the growth of primary tumors and impede metastasis (Mai et al., Medicinal Research Reviews, 25: 261-309,2005).

In view of the above, HDAC inhibitors can have great potential in the treatment of cell proliferative diseases or conditions, including tumours with mutated p53 genes. Patent application EP 1472216 published on August 14, 2003 discloses bicyclic hydroxamates as inhibitors of histone deacetylase.

Patent applications EP1485099, EP1485348, EP1485353, EP1485354, EP1485364, EP1485365, EP 1485370, EP 1485378 published on 18 September, 2003, amongst others, disclose substituted piperazinylpyrimidinylhydroxamic acids as inhibitors of histone deacetylase, furthermore EP 1485365 discloses R306465.

Patent application EP1492534 published on 9 October, 2003, discloses carbamic acid compounds comprising a piperazine linkage, as HDAC inhibitors.

Patent application EP1495002 published on 23 October, 2003, disclose substituted piperazinyl phenyl benzamide compounds, as histone deacetylase inhibitors.

Patent application WO03/092686 published on 13 November, 2003, discloses benzamides as histone deacetylase inhibitors.

Patent application WO04/009536 published on 29 January, 2004, discloses derivatives containing an alkyl linker between the aryl group and the hydroxamate, as histone deacetylase inhibitors.

Patent application EP1525199 published on 12 February, 2004, discloses (hetero)arylalkenyl substituted bicyclic hydroxamates, as histone deacetylase inhibitors.

Patent application EP 1572626 published on 24 June 2004, discloses arylene-carboxylic acid (2-amino-phenyl)-amide derivatives as pharmacological agents.

Patent application EP 1581484 published on 29 July 2004, discloses derivatives of N-hydroxy-benzamide derivatives with anti-inflammatory and antitumour activity.

Patent application EP1585735 published on 29 July 2004, discloses substituted aryl hydroxamate derivatives as histone deacetylase inhibitors.

Patent application EP1592667 published on 19 August 2004, discloses mono-acylated O-phenylendiamines derivatives as pharmacological agents.

Patent application EP 1590340 published on 19 August 2004, discloses diaminophenylene derivatives as histone deacetylase inhibitors.

Patent application EP1592665 published on 26 August 2004, discloses benzamide derivatives as histone deacetylase inhibitors.

Patent application WO04/072047 published on 26 August 2004, discloses indoles, benzimidazoles and naphhimidazoles as histone deacetylase inhibitors.

Patent application EP1608628 published on 30 September 2004, discloses hydroxamates linked to non-aromatic heterocyclic ring systems as histone deacetylase inhibitors.

Patent application EP 1613622 published on 14 October 2004, discloses oxime derivatives as histone deacetylase inhibitors.

Patent application EP 1611088 published on 28 October 2004, discloses hydroxamate derivatives as histone deacetylase inhibitors.

Patent application WO05/028447 published on 31 March 2005, discloses benzimidazoles as histone deacetylase inhibitors.

Patent applications WO05/030704 and WO05/030705 published on 7 April 2005, discloses benzamides as histone deacetylase inhibitors.

Patent application WO05/040101 published on 6 May 2005, disloses acylurea connected and sulfonylurea connected hydroxamates as histone deacetylase inhibitors.

Patent application WO05/040161 also published on 6 May 2005, discloses biaryl linked hydroxamates as histone deacetylase inhibitors.

Patent application WO05/075469 published on 18 August 2005, discloses thiazolyl hydroxamic acids and Thiadiazolyl hydroxamic acids as histone deacetylase inhibitors.

Patent application WO05/086898 published on 22 September 2005, discloses heteropentacyclic hydroxamic acids as histone deacetylase inhibitors.

Patent application WO05/092899 published on 6 October 2005, discloses alkenylbenzamides as histone deacetylases.

The compounds of the present invention differ from the prior art in structure, in their pharmacological activity and/or pharmacological potency.

The problem to be solved is to provide histone deacetylase inhibitors with high enzymatic and cellular activity that have increased bioavailability.

The novel compounds of the present invention solve the above-described problem. The compounds of the present invention show excellent histone deacetylase inhibiting enzymatic and cellular activity. They have a high capacity to activate the p21 gene. They can have a desirable pharmacokinetic profile and can have a low affinity for the P450 enzymes, which reduces the risk of adverse drug-drug interaction allowing also for a wider safety margin.

This invention concerns compounds of formula (I) 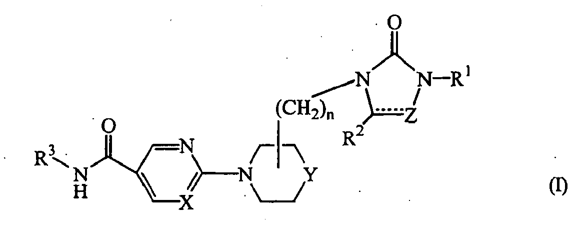 the *N*-oxide forms, the pharmaceutically acceptable addition salts and the stereochemically isomeric forms thereof, wherein
each X is independently N or CH;
each Y is independently O, CH or CH₂ and when Y is CH then the substituent is attached to the Y atom of the ring structure;
each Z is independently C=O, CH₂ or CH and when Z is CH then the dotted line is a bond;
n is 0 or 1 and when n is 0 then a direct bond is intended;
R¹ is phenyl, naphtalenyl, heterocyclyl, phenylC₁₋₆alkyl, naphtalenylC₁₋₆alkyl, heterocyclylC₁₋₆alkyl; wherein each of said phenyl, naphtalenyl or heterocyclyl is optionally substituted with one, two or three substituents each independently selected from hydrogen, halo, C₁₋₆alkyl, C₁₋₆alkyloxy, polyhaloC₁₋₆alkyl, phenyl, phenyloxy, cyano, C₁₋₆alkylcarbonylamino or two substituents taken together can form the bivalent radical -O-(CH₂)₂-O-(CH₂)₂- O-(CH₂)₂- O-(CH₂)₂- O-;
R² is hydrogen, C₁₋₆alkyl or phenyl wherein each phenyl is optionally substituted with one or two substituents each independently selected from hydrogen, halo, C₁₋₆alkyl, C₁₋₆alkyloxy, phenyloxy or cyano;
R³ is hydroxy or a radical of formula (a-1) 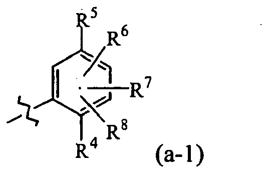 wherein
R⁴ is hydroxy or -NH₂;
R⁵ is hydrogen, thienyl, furanyl or phenyl and each thienyl, furanyl or phenyl can optionally be substituted with halo, amino, nitro, cyano, hydroxy, phenyl, C₁₋₆alkyl, (diC₁₋₆alkyl)amino, C₁₋₆alkyloxy, phenylC₁₋₆alkyloxy, hydroxyC₁₋₆alkyl, C₁₋₆alkyloxycarbonyl, hydroxycarbonyl, C₁₋₆alkylcarbonyl, polyhaloC₁₋₆alkyloxy, polyhaloC₁₋₆alkyl, C₁₋₆alkylsulfonyl, hydroxycarbonylC₁₋₆alkyl, C₁₋₆alkylcarbonylamino, aminosulfonyl, aminosulfonylC₁₋₆alkyl, isoxazolyl, aminocarbonyl, phenylC₂₋₆alkenyl, phenylC₃₋₆alkynyl or pyridinylC₃₋₆alkynyl; R⁶, R⁷ and R⁸ are each independently hydrogen, -NH₂, nitro, furanyl, halo, C₁₋₆alkyl, C₁₋₆alkyloxy, trifluoromethyl, thienyl, phenyl, C₁₋₆alkylcarbonylamino, aminocarbonylC₁₋₆alkyl or -C=C-CH₂-R⁹; wherein R⁹ is hydrogen, C₁₋₆alkyl, hydroxy, amino or C₁₋₆alkyloxy; and
heterocyclyl in the above is furanyl, thienyl, pyrrolyl, pyrrolinyl, pyrolidinyl, dioxolyl, oxazolyl, thiazolyl, imidazolyl, imidazolinyl, imidazolidinyl, pyrazolyl, pyrazolinyl, pyrazolidinyl, isoxazolyl, isothiazolyl, oxadiazolyl, triazolyl, thiadiazolyl, pyranyl, pyridinyl, piperidinyl, dioxanyl, morpholinyl, dithianyl, thiomorpholinyl, pyridazinyl, pyrimidinyl, pyrazinyl, piperazinyl, triazinyl, trithianyl, indolizinyl, indolyl, indolinyl, benzofuranyl, benzothiophenyl, indazolyl, benzimidazolyl, benzthiazolyl, purinyl, quinolizinyl, quinolinyl, cinnolinyl, phthalazinyl, quinazolinyl, quinoxalinyl or naphtyridinyl.

Lines drawn into ring systems from substituents indicate that the bond may be attached to any of the suitable ring atoms of the ring system.

The term "histone deacetylase inhibitor" or "inhibitor of histone deacetylase" is used to identify a compound, which is capable of interacting with a histone deacetylase and inhibiting its activity, more particularly its enzymatic activity. Inhibiting histone deacetylase enzymatic activity means reducing the ability of a histone deacetylase to remove an acetyl group from a histone. Preferably, such inhibition is specific, i.e. the histone deacetylase inhibitor reduces the ability of a histone deacetylase to remove an acetyl group from a histone at a concentration that is lower than the concentration of the inhibitor that is required to produce some other, unrelated biological effect.

As used in the foregoing definitions and hereinafter, halo is generic to fluoro, chloro, bromo and iodo; C₁₋₄alkyl defines straight and branched chain saturated hydrocarbon radicals having from 1 to 4 carbon atoms such as, e.g. methyl, ethyl, propyl, butyl, 1-methylethyl, 2-methylpropyl and the like; C₁₋₆alkyl includes C₁₋₄alkyl and the higher homologues thereof having 5 to 6 carbon atoms such as, for example, pentyl, 2-methylbutyl, hexyl, 2-methylpentyl and the like; C₂₋₆alkenyl defines straight and branched chain hydrocarbon radicals containing one double bond and having from 2 to 6 carbon atoms such as, for example, ethenyl, 2-propenyl, 3-butenyl, 2-pentenyl, 3-pentenyl, 3-methyl-2-butenyl, and the like; C₃₋₆alkynyl defines straight and branch chained hydrocarbon radicals containing one triple bond and having from 3 to 6 carbon atoms, such as, for example, 2-propynyl, 3-butynyl, 2-butynyl, 2-pentynyl, 3-pentynyl, 3-hexynyl, and the like; polyhaloC₁₋₆alkyl defines C₁₋₆alkyl containing three identical or different halo substituents for example trifluoromethyl; and C₃₋₆cycloalkyl includes cyclic hydrocarbon groups having from 3 to 6 carbons, such as cyclopropyl, cyclobutyl, cyclopentyl, cyclopentenyl, cyclohexyl and the like.

Pharmaceutically acceptable addition salts encompass pharmaceutically acceptable acid addition salts and pharmaceutically acceptable base addition salts. The pharmaceutically acceptable acid addition salts as mentioned hereinabove are meant to comprise the therapeutically active non-toxic acid addition salt forms, which the compounds of formula (I) are able to form. The compounds of formula (I) which have basic properties can be converted in their pharmaceutically acceptable acid addition salts by treating said base form with an appropriate acid. Appropriate acids comprise, for example, inorganic acids such as hydrohalic acids, e.g. hydrochloric or hydrobromic acid; sulfuric; nitric; phosphoric and the like acids; or organic acids such as, for example, acetic, trifluoroacetic, propanoic, hydroxyacetic, lactic, pyruvic, oxalic, malonic, succinic (*i.e*. butanedioic acid), maleic, fumaric, malic, tartaric, citric, methanesulfonic, ethanesulfonic, benzenesulfonic, *p*-toluenesulfonic, cyclamic, salicylic, *p*-amino-salicylic, pamoic and the like acids.

The compounds of formula (I) which have acidic properties may be converted in their pharmaceutically acceptable base addition salts by treating said acid form with a suitable organic or inorganic base. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, *N*-methyl-D-glucamine, hydrabamine salts, and salts with amino acids such as, for example, arginine, lysine and the like.

The term "acid or base addition salts" also comprises the hydrates and the solvent addition forms, which the compounds of formula (I) are able to form. Examples of such forms are e.g. hydrates, alcoholates and the like.

The term "stereochemically isomeric forms of compounds of formula (I)", as used herein, defines all possible compounds made up of the same atoms bonded by the same sequence of bonds but having different three-dimensional structures, which are not interchangeable, which the compounds of formula (I) may possess. Unless otherwise mentioned or indicated, the chemical designation of a compound encompasses the mixture of all possible stereochemically isomeric forms, which said compound may possess. Said mixture may contain all diastereomers and/or enantiomers of the basic molecular structure of said compound. All stereochemically isomeric forms of the compounds of formula (I) both in pure form or in admixture with each other are intended to be embraced within the scope of the present invention.

The N-oxide forms of the compounds of formula (I) are meant to comprise those compounds of formula (I) wherein one or several nitrogen atoms are oxidized to the so-called *N*-oxide, particularly those *N*-oxides wherein one or more of the piperidine-, piperazine or pyridazinyl-nitrogens are *N*-oxidized.

Some of the compounds of formula (I) may also exist in their tautomeric forms. Such forms although not explicitly indicated in the above formula are intended to be included within the scope of the present invention.

Whenever used hereinafter, the term "compounds of formula (I)" is meant to include also the pharmaceutically acceptable addition salts and all stereoisomeric forms.

As used herein, the terms "histone deacetylase" and "HDAC" are intended to refer to any one of a family of enzymes that remove acetyl groups from the ε-amino groups of lysine residues at the N-terminus of a histone. Unless otherwise indicated by context, the term "histone" is meant to refer to any histone protein, including H1, H2A, H2B, H3, H4, and H5, from any species. Human HDAC proteins or gene products, include, but are not limited to, HDAC-1, HDAC-2, HDAC-3, HDAC-4, HDAC-5, HDAC-6, HDAC-7, HDAC-8, HDAC-9, HDAC-10 and HDAC-11. The histone deacetylase can also be derived from a protozoal or fungal source.

A first group of interesting compounds consists of those compounds of formula (I) wherein one or more of the following restrictions apply:
a) each X is N;
b) each Y is independently O or CH;
c) n is 1;
d) R¹ is phenyl, naphtalenyl or heterocyclylC₁₋₆alkyl wherein each of said phenyl, naphtalenyl or heterocyclylC₁₋₆alkyl is optionally substituted with one, two or three substituents each independently selected from hydrogen, halo, C₁₋₆alkyl, C₁₋₆alkyloxy, polyhaloC₁₋₆alkyl, phenyl, phenyloxy, or cyano, or two substituents taken together can form the bivalent radical -O-(CH₂)₂-O-(CH₂)₂- O-(CH₂)₂- O-(CH₂)₂-O-;
e) R² is hydrogen, C₁₋₆alkyl or phenyl; and
f) R³ is hydroxy.

A second group of interesting compounds consists of those compounds of formula (I) wherein one or more of the following restrictions apply:
a) each X is N;
b) each Y is CH;
c) each Z is CH;
c) R¹ is phenyl or heterocyclylC₁₋₆alkyl wherein each of said phenyl or heterocyclylC₁₋₆alkyl is optionally substituted with one, two or three substituents each independently selected from hydrogen, C₁₋₆-alkyl, phenyl or phenyloxy;
d) R² is hydrogen; and
e) R³ is hydroxy.

A third group of interesting compounds consists of those compounds of formula (I) wherein one or more of the following restrictions apply:
a) R³ is a radical of formula (a-1);
b) R⁴ is -NH₂;
c) R⁵ is hydrogen or thienyl; and
d) R⁶, R⁷ and R⁸ are each independently hydrogen.

A group of preferred compounds consists of those compounds of formula (I) wherein each X is N; each Y is independently O or CH; n is 1 ; R¹ is phenyl, naphtalenyl or heterocyclylC₁₋₆alkyl wherein each of said phenyl, naphtalenyl or heterocyclylC₁₋₆alkyl is optionally substituted with one, two or three substituents each independently selected from hydrogen, halo, C₁₋₆alkyl, C₁₋₆alkyloxy, polyhaloC₁₋₆alkyl, phenyl, phenyloxy, or cyano, or two substituents taken together can form the bivalent radical -O-(CH₂)₂-O-(CH₂)₂- O-(CH₂)₂- O-(CH₂)₂- O-; R² is hydrogen, C₁₋₆alkyl or phenyl and R³ is hydroxyl.

A group of more preferred compounds consists of those compounds of formula (I) wherein each X is N; each Y is CH; each Z is CH; R¹ is phenyl or heterocyclylC₁₋₆alkyl wherein each of said phenyl or heterocyclylC₁₋₆alkyl is optionally substituted with one, two or three substituents each independently selected from hydrogen, C₁₋₆alkyl, phenyl or phenyloxy; R² is hydrogen and R³ is hydroxy.

The most preferred compounds are compound No.3, compound No.2 and compound No. 35.

| | |
|---|---|
| 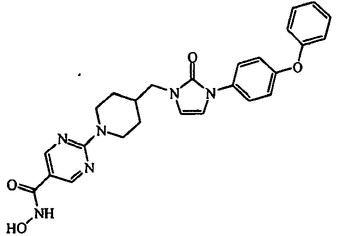 | 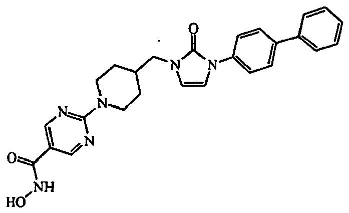 |
| Co. No. 2 | Co. No. 3 |
| 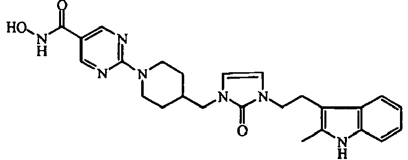 | |
| Co. No. 35 | |

The compounds of formula (I) and their pharmaceutically acceptable salts and N-oxides and stereochemically isomeric forms thereof may be prepared in conventional manner. The starting materials and some of the intermediates are known compounds and are commercially available or may be prepared according to conventional reaction procedures generally known in the art.

Some preparation methods will be described hereinafter in more detail. Other methods for obtaining final compounds of formula (I) are described in the examples.

Compounds of formula (I), wherein R³ is hydroxy, herein referred to as compounds of formula (I-a) may be prepared by reacting an intermediate of formula (II) with an appropriate acid, such as for example, trifluoro acetic acid. Said reaction is performed in an appropriate solvent, such as, for example, methanol or dichloromethane.

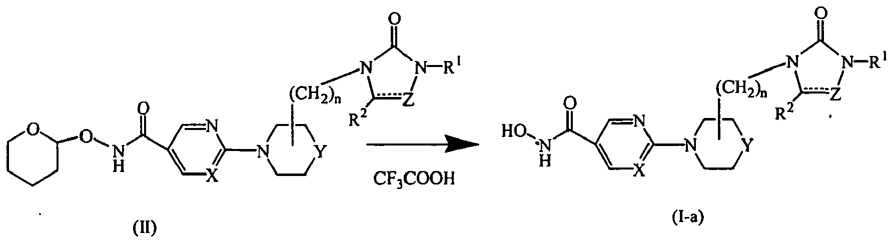

Compounds of formula (I) wherein R³ is a radical of formula (a-1) and R⁴ is -NH₂, herein referred to as compounds of formula (I-b) may be prepared by reacting an intermediate of formula (XV) with tin(II) chloride hydrate. Said reaction can be performed in an appropriate solvent, such as, for example, a mixture of tetrahydrofuran, methanol and water. Alternatively, compounds of formula (I-b) may be prepared by reacting an intermediate of formula (XV) with hydrogen in the presence of 10% palladium on charcoal in a suitable solvent such as for example methanol.

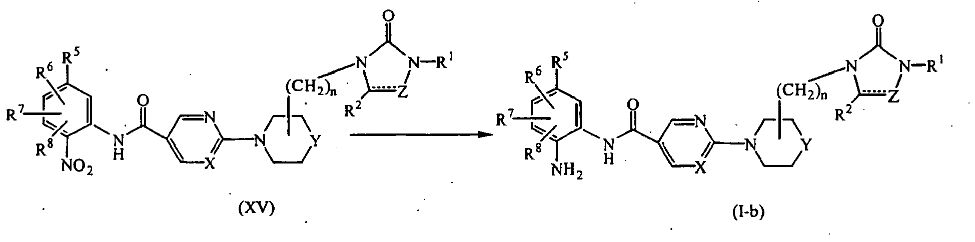

Compounds of formula (I-b) may also be prepared by reacting an intermediate of formula (XVI) with an appropriate acid, such as for example, trifluoro acetic acid. Said reaction is performed in an appropriate solvent, such as, for example, methanol or dichloromethane.

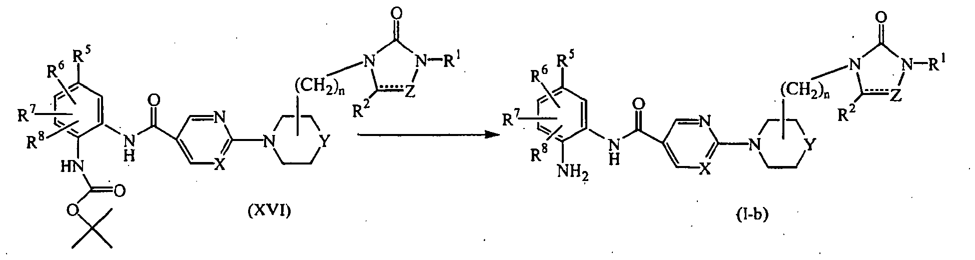

Compounds of formula (I), wherein R³ is a radical of formula (a-1) and R⁴ is hydroxy, herein referred to as compounds of formula (I-c) may be prepared by reacting an intermediate of formula (XVII) with tetrabutylammonium fluoride in an appropriate solvent such as, for example tetrahydrofuran. TBDMS in the intermediate of formula (XVII) means *tert*-butyl(dimethyl)silanyl.

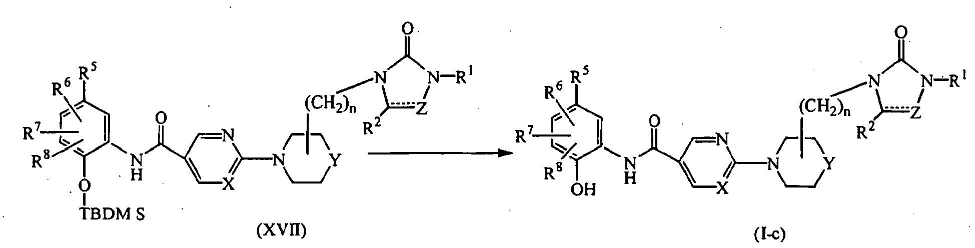

Intermediates of formula (II) may be prepared by reacting an intermediate of formula (III) with an intermediate of formula (IV) in the presence of appropriate reagents such as (3-dimethylamino-propy)1-ethyl-carbodiimide) hydrochloride (EDC) and 1-hydroxy-1*H*-benzotriazole (HOBT). The reaction may be performed in the presence of a base such as triethylamine, in a suitable solvent, such as, a mixture of dichloromethane, *N*,*N-*dimethylformamide and tetrahydrofuran or a mixture of dichloromethane and tetrahydrofuran.

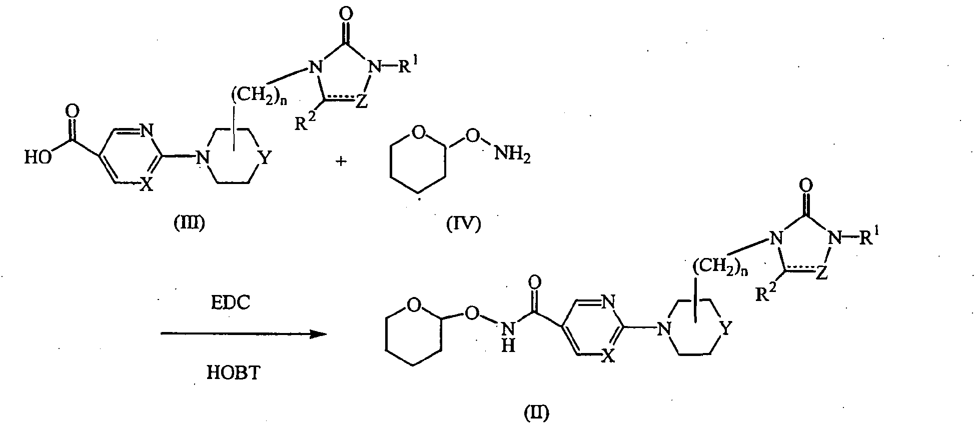

Intermediates of formula (XV) may be prepared by reacting an intermediate of formula (III) with an appropriate nitrophenylamine of formula (XVIII) in the presence of benzotriazol-1-yloxytris(dimethylamino)phosphoniumhexafluorophosphate (BOP) and sodium hydride. Said reaction can be performed in a suitable solvent such as, for example, pyridine.

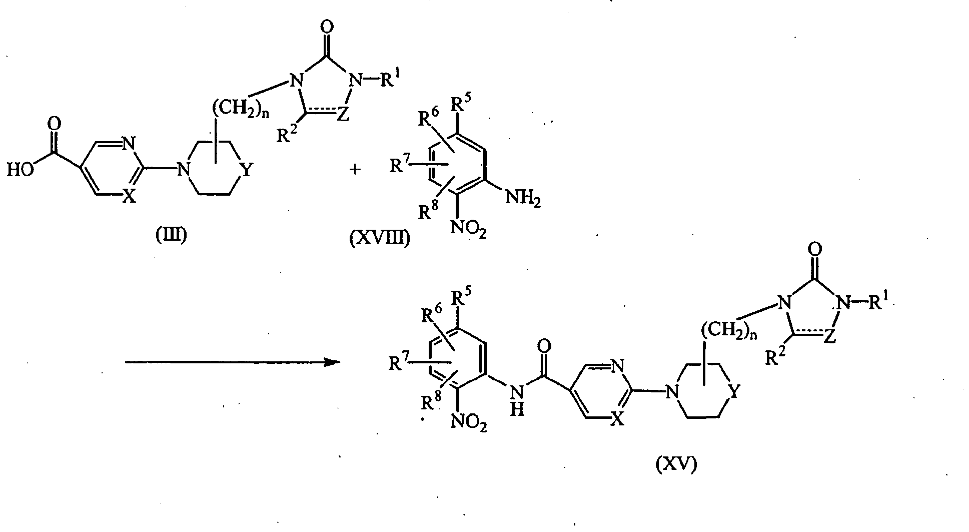

Also intermediates of formula (XVI) may be prepared by reacting an intermediate of formula (III) with an appropriate *tert*-butyloxycarbonyl (Boc) protected phenylamine of formula (XIX) in the presence of benzotriazol-1-yloxytris(dimethylamino)phosphoniumhexafluorophosphate (BOP) and sodium hydride. Said reaction can be performed in a suitable solvent such as, for example, pyridine.

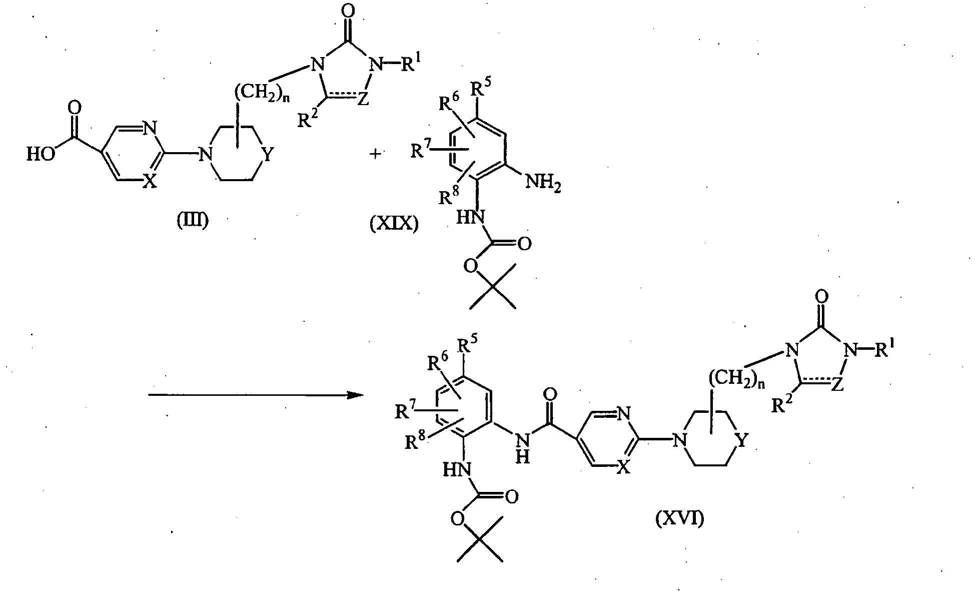

Intermediates of formula (XVII) may be prepared by reacting an intermediate of formula (III) with an appropriate *tert*-butyl(dimethyl)silanyl (TBDMS) protected phenylamine of formula (XX) in the presence of benzotriazol-1-yloxytris(dimethylamino)phosphoniumhexafluorophosphate (BOP) and triethylamine. Said reaction is performed in a suitable solvent such as, for example, N,N-dimethylformamide.

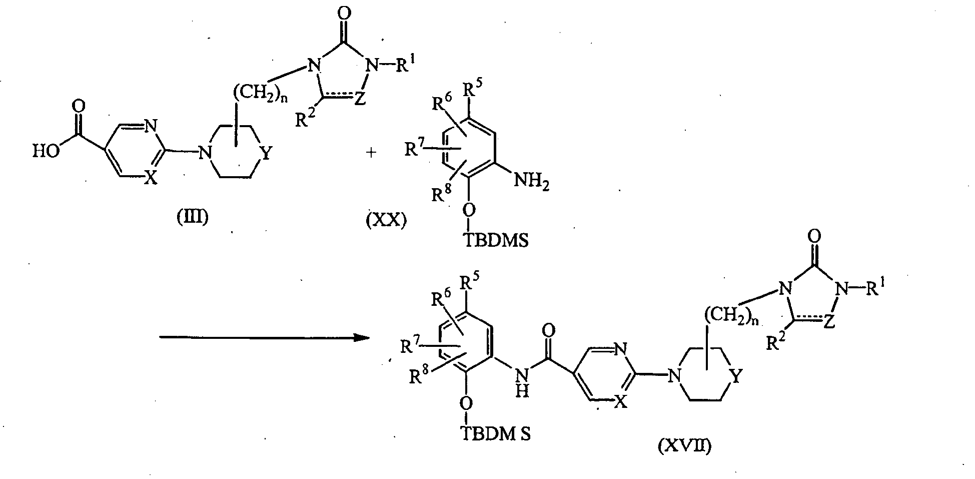

Intermediates of formula (II), wherein Z is C=O and R² is C₁₋₆alkyl (see drawing) or phenyl, herein referred to as intermediates of formula (II-a), can be prepared by reacting an intermediate of formula (VI) wherein R² is C₁₋₆alkyl (see drawing) or phenyl with an intermediate of formula (VII) in the presence of a suitable solvent, such as tetrahydrofuran.

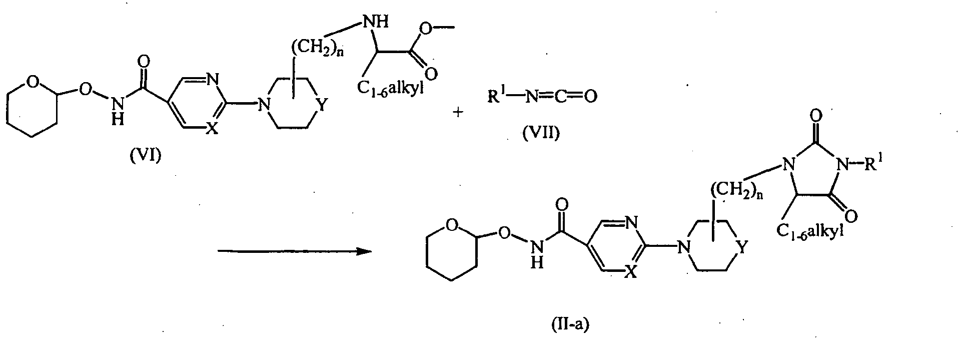

Intermediates of formula (III) may be prepared by reacting an intermediate of formula (V) with an appropriate acidic solution, e.g. hydrochloric acid, or basic solution, e.g. lithium hydroxide or sodiumhydroxide, in a suitable solvent such as dioxane, or a mixture of suitable solvents such as alcohols, acetonitrile and water.

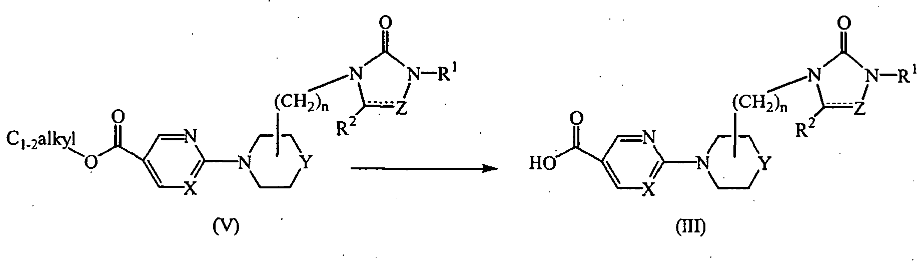

Intermediates of formula (V) wherein Z is CH₂ and R² is hydrogen, herein referred to as intermediates of formula (V-a) can be prepared by converting intermediates of formula (V), wherein Z is CH and the dotted line represents a bond, herein referred to as intermediates of formula (V-b), by catalytic hydrogenation of the intermediate of formula (V-b) with hydrogen in the presence of a catalyst, such as, for example, palladium on carbon (10%). The reaction may be performed in the presence of a base such as triethylamine, in a suitable solvent, such as tetrahydrofuran.

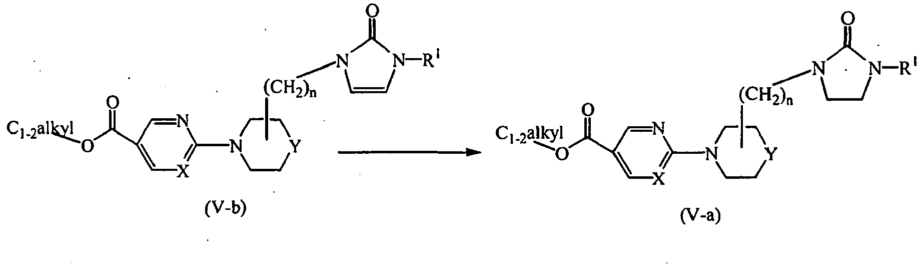

Intermediates of formula (VI) wherein R² is C₁₋₆alkyl (see drawing) or phenyl can be prepared by reacting intermediates of formula (VIII) with intermediates of formula (IX), wherein R² is C₁₋₆alkyl (see drawing) or phenyl and halo is f.e. chloro or bromo, in the presence of potassium carbonate and in the presence of a suitable solvent such as acetonitrile.

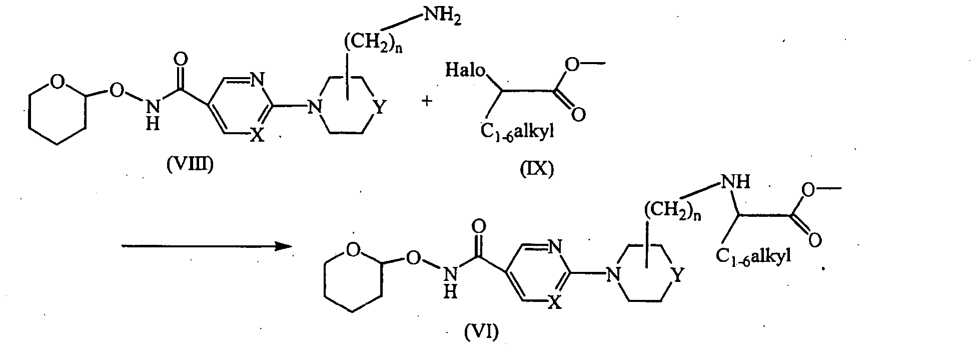

Intermediates of formula (V), wherein Z is C=O and R² is C₁₋₆alkyl or phenyl (see drawing), herein referred to as intermediates of formula (V-c), can be prepared by reacting an intermediate of formula (X), wherein R² is C₁₋₆alkyl or phenyl (see drawing) with an intermediate of formula (VII) in the presence of a suitable solvent, such as tetrahydrofuran.

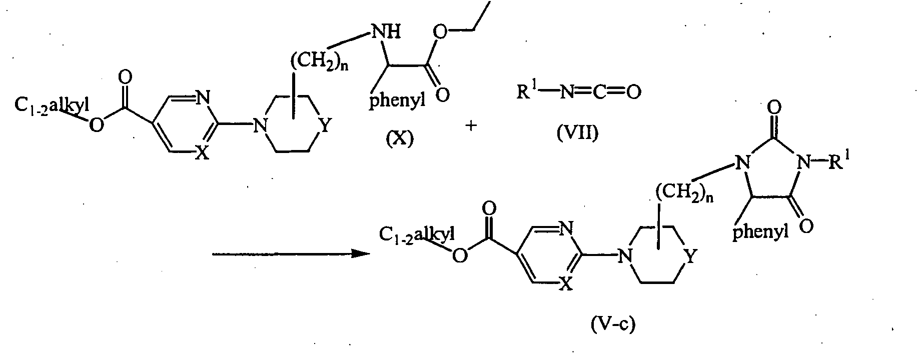

Intermediates of formula (X), wherein R² is C₁₋₆alkyl or phenyl (see drawing), can be prepared by reacting intermediates of formula (XI) with intermediates of formula (XII) wherein R² is C₁₋₆alkyl or phenyl (see drawing) and halo is f.e. chloro or bromo, in the presence of a suitable reagent such as potassium carbonate and in the presence of a suitable solvent such as acetonitrile.

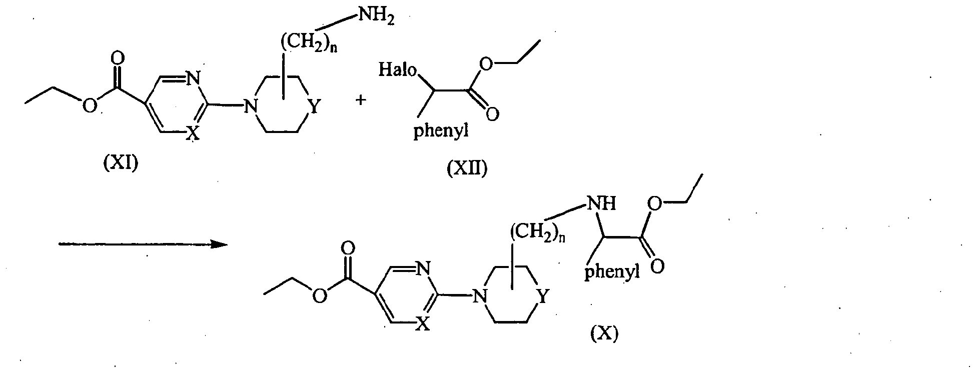

Intermediates of formula (V-b) can be prepared by converting intermediates of formula (XIII) in the presence of a suitable acid such as formic acid or hydrochloric acid in a suitable solvent such as methanol.

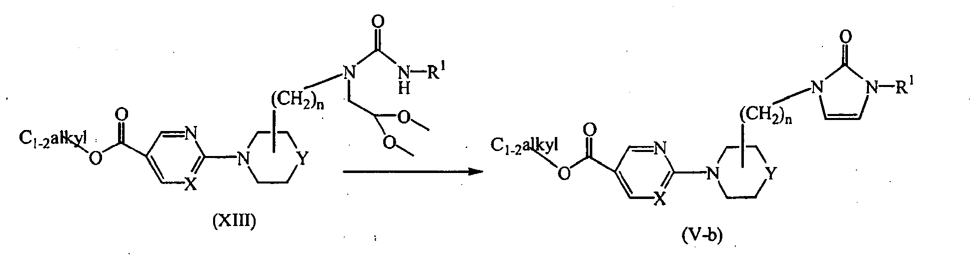

Intermediates of formula (XIII) can be prepared by reacting an intermediate of formula (XIV) with an intermediate of formula (VII) in the presence of a base such as triethylamine, in a suitable solvent, such as, dichloromethane or tetrahydrofuran. 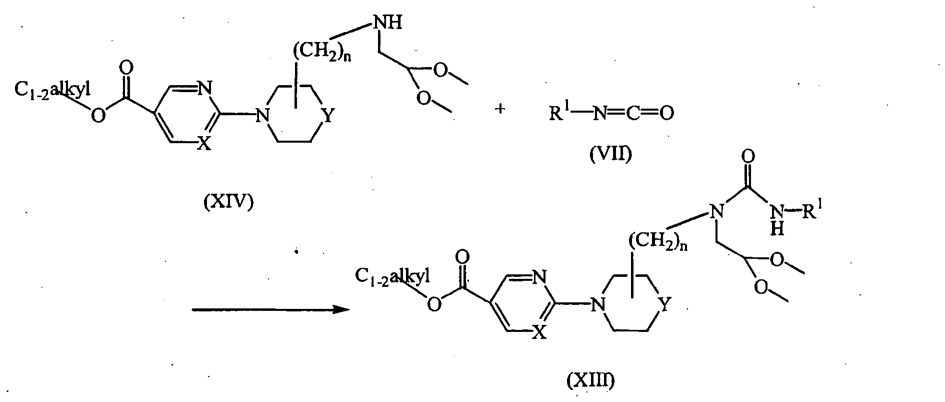 Intermediates of formula (XIV) can be prepared by reacting intermediates of formula (XI) with glyoxal dimethyl acetal and sodium triacetoxyborohydride, in the presence of a suitable solvent, such as for example tetrahydrofuran or with 2- bromo-1,1,diethoxyethane and potassium carbonate in the presence of a suitable solvent such as for example acetonitrile.

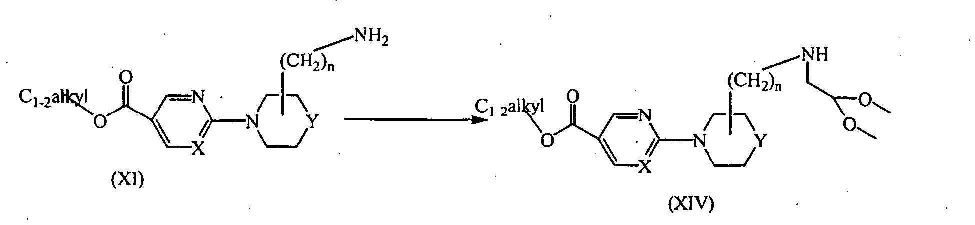

Intermediates of formula (XVIII) can be prepared by reacting an intermediate of formula (XXI), wherein W is a suitable leaving group such as, for example, bromo, with an appropriate boronic acid of formula (XXII), in the presence of tri-o-tolylphosphine and potassium carbonate. Said reaction can be performed in a suitable solvent such as, for example, dimethylether in the presence of a base such as potassium carbonate.

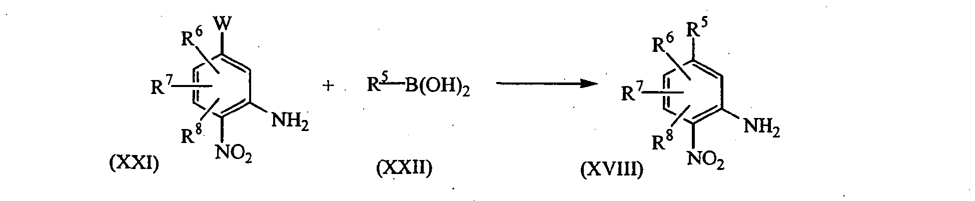

Intermediates of formula (XXI) can be prepared by reacting the appropriate nitrobenzene of formula (XXIII) with O-methyl-hydroxylamine hydrochloride, in the presence of potassium *tert*-butoxide and copper(I) chloride. Said reaction can be performed in a suitable solvent such as, dimethylether.

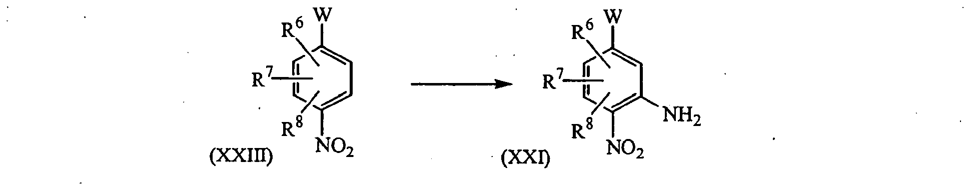

Intermediates of formula (XIX) can be prepared by reacting intermediates of formula (XXIV) with hydrogen in the presence of 10 % palladium on charcoal in a suitable solvent such as, for example methanol.

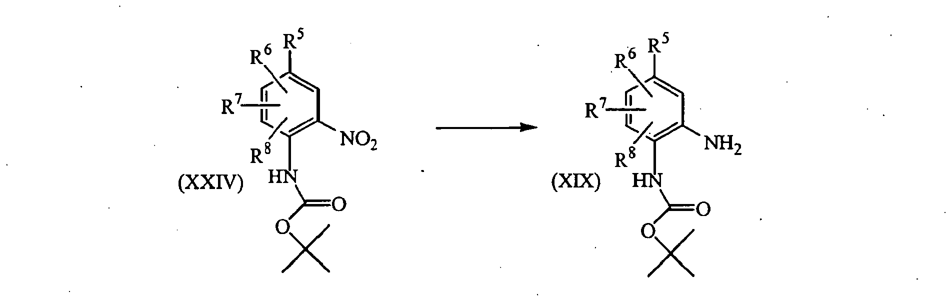

Intermediates of formula (XXIV) can be prepared by reacting the appropriate tert-butyl nitrophenyl carbamate or formula (XXV), wherein W is an appropriate leaving group, such as, for example bromine, with the boronic acid of formula (XXII) in the presence of tetrakis(triphenylphosphine)palladium and sodium carbonate. Said reaction can be performed in a suitable solvent such as for example, a mixture of dimethylether and water.

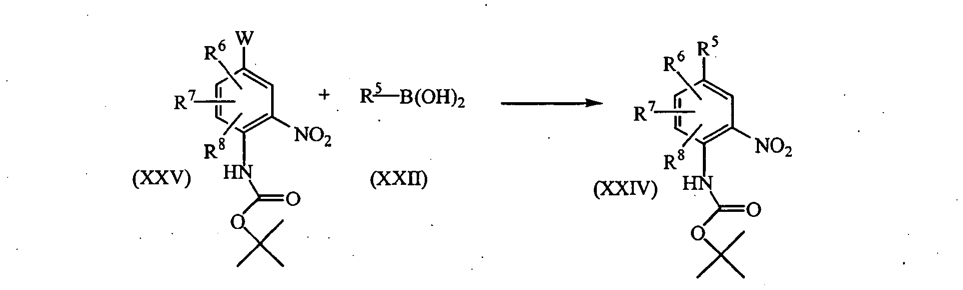

Intermediates of formula (XXV) can be prepared by reacting the appropriate nitroaniline of formula (XXVI) with tertiary butoxy carbonyl anhydride, in the presence of sodium hydride and in a suitable solvent such as, for example, *N*,*N*-dimethylformamide.

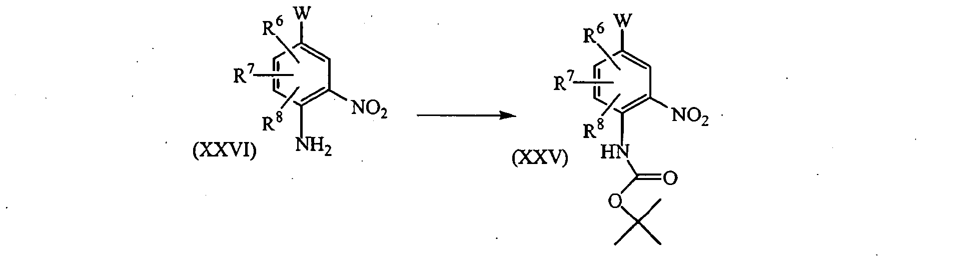

Intermediates of formula (XX) can be prepared by reacting the appropriate aminophenol of formula (XXVII) with *tert*-butyl-chloro-dimethyl-silane, in the presence of triethylamine in a suitable solvent such as tetrahydrofuran.

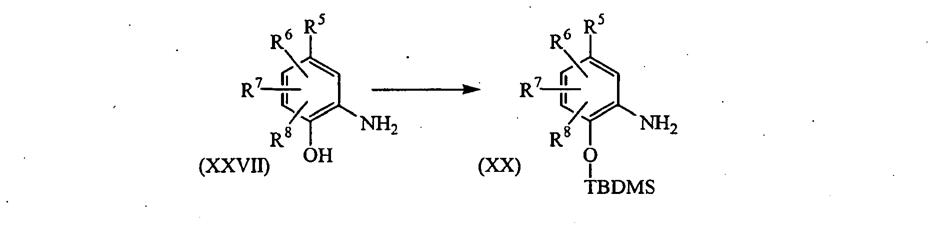

The compounds of formula (I) and some of the intermediates may have at least one stereogenic centre in their structure. This stereogenic centre may be present in an R or an S configuration.

The compounds of formula (I) as prepared in the hereinabove described processes are generally racemic mixtures of enantiomers, which can be separated from one another following art-known resolution procedures. The racemic compounds of formula (I) may be converted into the corresponding diastereomeric salt forms by reaction with a suitable chiral acid. Said diastereomeric salt forms are subsequently separated, for example, by selective or fractional crystallization and the enantiomers are liberated there from by alkali. An alternative manner of separating the enantiomeric forms of the compounds of formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

The compounds of formula (I), the pharmaceutically acceptable acid addition salts and stereoisomeric forms thereof have valuable pharmacological properties in that they have a histone deacetylase (HDAC) inhibitory effect.

This invention provides a method for inhibiting the abnormal growth of cells, including transformed cells, by administering an effective amount of a compound of the invention. Abnormal growth of cells refers to cell growth independent of normal regulatory mechanisms (e.g. loss of contact inhibition). This includes the inhibition of tumour growth both directly by causing growth arrest, terminal differentiation and/or apoptosis of cancer cells, and indirectly, by inhibiting neovascularization of tumours.

This invention also provides a method for inhibiting tumour growth by administering an effective amount of a compound of the present invention, to a subject, e.g. a mammal (and more particularly a human) in need of such treatment. In particular, this invention provides a method for inhibiting the growth of tumours by the administration of an effective amount of the compounds of the present invention. Examples of tumours which may be inhibited, but are not limited to, lung cancer (e.g. adenocarcinoma and including non-small cell lung cancer), pancreatic cancers (e.g. pancreatic carcinoma such as, for example exocrine pancreatic carcinoma), colon cancers (e.g. colorectal carcinomas, such as, for example, colon adenocarcinoma and colon adenoma), prostate cancer including the advanced disease, hematopoietic tumours of lymphoid lineage (e.g. acute lymphocytic leukemia, B-cell lymphoma, Burkitt's lymphoma), myeloid leukemias (for example, acute myelogenous leukemia (AML)), thyroid follicular cancer, myelodysplastic syndrome (MDS), tumours of mesenchymal origin (e.g. fibrosarcomas and rhabdomyosarcomas), melanomas, teratocarcinomas, neuroblastomas, gliomas, benign tumour of the skin (e.g. keratoacanthomas), breast carcinoma (e.g. advanced breast cancer), kidney carcinoma, ovary carcinoma, bladder carcinoma and epidermal carcinoma.

The compound according to the invention may be used for other therapeutic purposes, for example:
a) the sensitisation of tumours to radiotherapy by administering the compound according to the invention before, during or after irradiation of the tumour for treating cancer;
b) treating arthropathies and osteopathological conditions such as rheumatoid arthritis, osteoarthritis, juvenile arthritis, gout, polyarthritis, psoriatic arthritis, ankylosing spondylitis and systemic lupus erythematosus;
c) inhibiting smooth muscle cell proliferation including vascular proliferative disorders, atherosclerosis and restenosis;
d) treating inflammatory conditions and dermal conditions such as ulcerative colitis, Crohn's disease, allergic rhinitis, graft vs. host disease, conjunctivitis, asthma, ARDS, Behcets disease, transplant rejection, uticaria, allergic dermatitis, alopecia areata, scleroderma, exanthema, eczema, dermatomyositis, acne, diabetes, systemic lupus erythematosis, Kawasaki's disease, multiple sclerosis, emphysema, cystic fibrosis and chronic bronchitis;
e) treating endometriosis, uterine fibroids, dysfunctional uterine bleeding and endometrial hyperplasia;
f) treating ocular vascularisation including vasculopathy affecting retinal and choroidal vessels;
g) treating a cardiac dysfunction;
h) inhibiting immunosuppressive conditions such as the treatment of HIV infections;
i) treating renal dysfunction;
j) suppressing endocrine disorders;
k) inhibiting dysfunction of gluconeogenesis;
l) treating a neuropathology for example Parkinson's disease or a neuropathology that results in a cognitive disorder, for example, Alzheimer's disease or polyglutamine related neuronal diseases;
m) treating psychiatric disorders for example schizophrenia, bipolar disorder, depression, anxiety and psychosis;
n) inhibiting a neuromuscular pathology, for example, amylotrophic lateral sclerosis;
o) treating spinal muscular atrophy;
p) treating other pathologic conditions amenable to treatment by potentiating expression of a gene;
q) enhancing gene therapy;
r) inhibiting adipogenesis;
s) treating parasitosis such as malaria.

Hence, the present invention discloses the compounds of formula (I) for use as a medicine as well as the use of these compounds of formula (I) for the manufacture of a medicament for treating one or more of the above mentioned conditions.

The compounds of formula (I), the pharmaceutically acceptable acid addition salts and stereoisomeric forms thereof can have valuable diagnostic properties in that they can be used for detecting or identifying a HDAC in a biological sample comprising detecting or measuring the formation of a complex between a labelled compound and a HDAC.

The detecting or identifying methods can use compounds that are labelled with labelling agents such as radioisotopes, enzymes, fluorescent substances, luminous substances, etc. Examples of the radioisotopes include ¹²⁵I, ¹³¹I, ³H and ¹⁴C. Enzymes are usually made detectable by conjugation of an appropriate substrate which, in turn catalyses a detectable reaction. Examples thereof include, for example, beta-galactosidase, beta-glucosidase, alkaline phosphatase, peroxidase and malate dehydrogenase, preferably horseradish peroxidase. The luminous substances include, for example, luminol, luminol derivatives, luciferin, aequorin and luciferase.

Biological samples can be defined as body tissue or body fluids. Examples of body fluids are cerebrospinal fluid, blood, plasma, serum, urine, sputum, saliva and the like.

In view of their useful pharmacological properties, the subject compounds may be formulated into various pharmaceutical forms for administration purposes.

To prepare the pharmaceutical compositions of this invention, an effective amount of a particular compound, in base or acid addition salt form, as the active ingredient is combined in intimate admixture with a pharmaceutically acceptable carrier, which carrier may take a wide variety of forms depending on the form of preparation desired for administration. These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets.

Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, to aid solubility for example, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Said additives may facilitate the administration to the skin and/or may be helpful for preparing the desired compositions. These compositions may be administered in various ways, e.g., as a transdermal patch, as a spot-on or as an ointment.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient, calculated to produce the desired therapeutic effect, in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

Those skilled in the art could easily determine the effective amount from the test results presented hereinafter. In general it is contemplated that a therapeutically effective amount would be from 0.005 mg/kg to 100 mg/kg body weight, and in particular from 0.005 mg/kg to 10 mg/kg body weight. It may be appropriate to administer the required dose as two, three, four or more sub-doses at appropriate intervals throughout the day. Said sub-doses may be formulated as unit dosage forms, for example, containing 0.5 to 500 mg, and in particular 10 mg to 500 mg of active ingredient per unit dosage form.

As another aspect of the present invention a combination of a HDAC-inhibitor with another anticancer agent is envisaged, especially for use as a medicine, more specifically in the treatment of cancer or related diseases.

For the treatment of the above conditions, the compounds of the invention may be advantageously employed in combination with one or more other medicinal agents, more particularly, with other anti-cancer agents. Examples of anti-cancer agents are:
- platinum coordination compounds for example cisplatin, carboplatin or oxalyplatin;
- taxane compounds for example paclitaxel or docetaxel;
- topoisomerase I inhibitors such as camptothecin compounds for example irinotecan or topotecan;
- topoisomerase II inhibitors such as anti-tumour podophyllotoxin derivatives for example etoposide or teniposide;
- anti-tumour vinca alkaloids for example vinblastine, vincristine or vinorelbine;
- anti-tumour nucleoside derivatives for example 5-fluorouracil, gemcitabine or capecitabine;
- alkylating agents such as nitrogen mustard or nitrosourea for example cyclophosphamide, chlorambucil, carmustine or lomustine;
- anti-tumour anthracycline derivatives for example daunorubicin, doxorubicin, idarubicin or mitoxantrone;
- HER2 antibodies for example trastuzumab;
- estrogen receptor antagonists or selective estrogen receptor modulators for example tamoxifen, toremifene, droloxifene, faslodex or raloxifene;
- aromatase inhibitors such as exemestane, anastrozole, letrazole and vorozole;
- differentiating agents such as retinoids, vitamin D and retinoic acid metabolism blocking agents (RAMBA) for example accutane;
- DNA methyl transferase inhibitors for example azacytidine;
- kinase inhibitors for example flavoperidol, imatinib mesylate or gefitinib;
- farnesyltransferase inhibitors;
- other HDAC inhibitors
- inhibitors of the ubiquitin-proteasome pathway for example Velcade; or
- Yondelis.

The term "platinum coordination compound" is used herein to denote any tumour cell growth inhibiting platinum coordination compound which provides platinum in the form of an ion.

The term "taxane compounds" indicates a class of compounds having the taxane ring system and related to or derived from extracts from certain species of yew (Taxus) trees.

The term "topisomerase inhibitors" is used to indicate enzymes that are capable of altering DNA topology in eukaryotic cells. They are critical for important cellular functions and cell proliferation. There are two classes of topoisomerases in eukaryotic cells, namely type I and type II. Topoisomerase I is a monomeric enzyme of approximately 100,000 molecular weight. The enzyme binds to DNA and introduces a transient single-strand break, unwinds the double helix (or allows it to unwind) and subsequently reseals the break before dissociating from the DNA strand. Topisomerase II has a similar mechanism of action which involves the induction of DNA strand breaks or the formation of free radicals.

The term "camptothecin compounds" is used to indicate compounds that are related to or derived from the parent camptothecin compound which is a water-insoluble alkaloid derived from the Chinese tree Camptothecin acuminata and the Indian tree Nothapodytes foetida.

The term "podophyllotoxin compounds" is used to indicate compounds that are related to or derived from the parent podophyllotoxin, which is extracted from the mandrake plant.

The term "anti-tumour vinca alkaloids" is used to indicate compounds that are related to or derived from extracts of the periwinkle plant (Vinca rosea).

The term "alkylating agents" encompass a diverse group of chemicals that have the common feature that they have the capacity to contribute, under physiological conditions, alkyl groups to biologically vital macromolecules such as DNA. With most of the more important agents such as the nitrogen mustards and the nitrosoureas, the active alkylating moieties are generated *in vivo* after complex degradative reactions, some of which are enzymatic. The most important pharmacological actions of the alkylating agents are those that disturb the fundamental mechanisms concerned with cell proliferation in particular DNA synthesis and cell division. The capacity of alkylating agents to interfere with DNA function and integrity in rapidly proliferating tissues provides the basis for their therapeutic applications and for many of their toxic properties.

The term "anti-tumour anthracycline derivatives" comprise antibiotics obtained from the fungus *Strep. peuticus var. caesius* and their derivatives, characterised by having a tetracycline ring structure with an unusual sugar, daunosamine, attached by a glycosidic linkage.

Amplification of the human epidermal growth factor receptor 2 protein (HER 2) in primary breast carcinomas has been shown to correlate with a poor clinical prognosis for certain patients. Trastuzumab is a highly purified recombinant DNA-derived humanized monoclonal IgG1 kappa antibody that binds with high affiniity and specificity to the extracellular domain of the HER2 receptor.

Many breast cancers have estrogen receptors and growth of these tumours can be stimulated by estrogen. The terms "estrogen receptor antagonists" and "selective estrogen receptor modulators" are used to indicate competitive inhibitors of estradiol binding to the estrogen receptor (ER). Selective estrogen receptor modulators, when bound to the ER, induces a change in the three-dimensional shape of the receptor, modulating its binding to the estrogen responsive element (ERE) on DNA.

In postmenopausal women, the principal source of circulating estrogen is from conversion of adrenal and ovarian androgens (androstenedione and testosterone) to estrogens (estrone and estradiol) by the aromatase enzyme in peripheral tissues. Estrogen deprivation through aromatase inhibition or inactivation is an effective and selective treatment for some postmenopausal patients with hormone-dependent breast cancer.

The term "antiestrogen agent" is used herein to include not only estrogen receptor antagonists and selective estrogen receptor modulators but also aromatase inhibitors as discussed above.

The term "differentiating agents" encompass compounds that can, in various ways, inhibit cell proliferation and induce differentiation. Vitamin D and retinoids are known to play a major role in regulating growth and differentiation of a wide variety of normal and malignant cell types. Retinoic acid metabolism blocking agents (RAMBA's) increase the levels of endogenous retinoic acids by inhibiting the cytochrome P450-mediated catabolism of retinoic acids.

DNA methylation changes are among the most common abnormalities in human neoplasia. Hypermethylation within the promotors of selected genes is usually associated with inactivation of the involved genes. The term "DNA methyl transferase inhibitors" is used to indicate compounds that act through pharmacological inhibition of DNA methyl transferase and reactivation of tumour suppressor gene expression.

The term "kinase inhibitors" comprises potent inhibitors of kinases that are involved in cell cycle progression and programmed cell death (apoptosis)

The term "farnesyltransferase inhibitors" is used to indicate compounds that were designed to prevent farnesylation of Ras and other intracellular proteins. They have been shown to have effect on malignant cell proliferation and survival.

The term "other HDAC inhibitors" comprises but is not limited to:
- carboxylates for example butyrate, cinnamic acid, 4-phenylbutyrate or valproic acid;
- hydroxamic acids for example suberoylanilide hydroxamic acid (SAHA), piperazine containing SAHA analogues, biaryl hydroxamate A-161906 and its carbozolylether-, tetrahydropyridine- and tetralone- analogues, bicyclic aryl-N-hydroxycarboxamides, pyroxamide, CG-1521, PXD-101, sulfonamide hydroxamic acid, LAQ-824, LBH-589, trichostatin A (TSA), oxamflatin, scriptaid, scriptaid related tricyclic molecules, m-carboxy cinnamic acid bishydroxamic acid (CBHA), CBHA-like hydroxamic acids, trapoxin-hydroxamic acid analogue, CRA-024781, R306465 and related benzoyl- and heteroaryl-hydroxamic acids, aminosuberates and malonyldiamides;
- cyclic tetrapeptides for example trapoxin, apidicin, depsipeptide, spiruchostatin-related compounds, RedFK-228, sulfhydryl-containing cyclic tetrapeptides (SCOPs), hydroxamic acid containing cyclic tetrapeptides (CHAPs), TAN-174s and azumamides;
- benzamides for example MS-275 or CI-994, or
- depudecin.

The term "inhibitors of the ubiquitin-proteasome pathway" is used to identify compounds that inhibit the targeted destruction of cellular proteins in the proteasome, including cell cycle regulatory proteins.

For the treatment of cancer the compounds according to the present invention may be administered to a patient as described above, in conjunction with irradiation. Irradiation means ionising radiation and in particular gamma radiation, especially that emitted by linear accelerators or by radionuclides that are in common use today. The irradiation of the tumour by radionuclides can be external or internal.

The present invention also relates to a combination according to the invention of an anti-cancer agent and a HDAC inhibitor according to the invention.

The present invention also relates to a combination according to the invention for use in medical therapy for example for inhibiting the growth of tumour cells.

The present invention also relates to a combinations according to the invention for inhibiting the growth of tumour cells.

The present invention also relates to a method of inhibiting the growth of tumour cells in a human subject which comprises administering to the subject an effective amount of a combination according to the invention.

This invention further provides a method for inhibiting the abnormal growth of cells, including transformed cells, by administering an effective amount of a combination according to the invention.

The other medicinal agent and HDAC inhibitor may be administered simultaneously (e.g. in separate or unitary compositions) or sequentially in either order. In the latter case, the two compounds will be administered within a period and in an amount and manner that is sufficient to ensure that an advantageous or synergistic effect is achieved. It will be appreciated that the preferred method and order of administration and the respective dosage amounts and regimes for each component of the combination will depend on the particular other medicinal agent and HDAC inhibitor being administered, their route of administration, the particular tumour being treated and the particular host being treated. The optimum method and order of administration and the dosage amounts and regime can be readily determined by those skilled in the art using conventional methods and in view of the information set out herein.

The platinum coordination compound is advantageously administered in a dosage of 1 to 500 mg per square meter (mg/m²) of body surface area, for example 50 to 400 mg/m², particularly for cisplatin in a dosage of about 75 mg/m² and for carboplatin in about 300 mg/m² per course of treatment.

The taxane compound is advantageously administered in a dosage of 50 to 400 mg per square meter (mg/m²) of body surface area, for example 75 to 250 mg/m², particularly for paclitaxel in a dosage of about 175 to 250 mg/m² and for docetaxel in about 75 to 150 mg/m² per course of treatment.

The camptothecin compound is advantageously administered in a dosage of 0.1 to 400 mg per square meter (mg/m²) of body surface area, for example 1 to 300 mg/m², particularly for irinotecan in a dosage of about 100 to 350 mg/m² and for topotecan in about 1 to 2 mg/m² per course of treatment.

The anti-tumour podophyllotoxin derivative is advantageously administered in a dosage of 30 to 300 mg per square meter (mg/m²) of body surface area, for example 50 to 250mg/m², particularly for etoposide in a dosage of about 35 to 100 mg/m² and for teniposide in about 50 to 250 mg/m² per course of treatment.

The anti-tumour vinca alkaloid is advantageously administered in a dosage of 2 to 30 mg per square meter (mg/m²) of body surface area, particularly for vinblastine in a dosage of about 3 to 12 mg/m², for vincristine in a dosage of about 1 to 2 mg/m², and for vinorelbine in dosage of about 10 to 30 mg/m² per course of treatment.

The anti-tumour nucleoside derivative is advantageously administered in a dosage of 200 to 2500 mg per square meter (mg/m²) of body surface area, for example 700 to 1500 mg/m², particularly for 5-FU in a dosage of 200 to 500mg/m², for gemcitabine in a dosage of about 800 to 1200 mg/m² and for capecitabine in about 1000 to 2500 mg/m² per course of treatment.

The alkylating agents such as nitrogen mustard or nitrosourea is advantageously administered in a dosage of 100 to 500 mg per square meter (mg/m²) of body surface area, for example 120 to 200 mg/m², particularly for cyclophosphamide in a dosage of about 100 to 500 mg/m² for chlorambucil in a dosage of about 0.1 to 0.2 mg/kg, for carmustine in a dosage of about 150 to 200 mg/m², and for lomustine in a dosage of about 100 to 150 mg/m² per course of treatment.

The anti-tumour anthracycline derivative is advantageously administered in a dosage of 10 to 75 mg per square meter (mg/m²) of body surface area, for example 15 to 60 mg/m², particularly for doxorubicin in a dosage of about 40 to 75 mg/m², for daunorubicin in a dosage of about 25 to 45mg/m², and for idarubicin in a dosage of about 10 to 15 mg/m² per course of treatment.

Trastuzumab is advantageously administered in a dosage of 1 to 5 mg per square meter (mg/m²) of body surface area, particularly 2 to 4 mg/m² per course of treatment.

The antiestrogen agent is advantageously administered in a dosage of about 1 to 100 mg daily depending on the particular agent and the condition being treated. Tamoxifen is advantageously administered orally in a dosage of 5 to 50 mg, preferably 10 to 20 mg twice a day, continuing the therapy for sufficient time to achieve and maintain a therapeutic effect. Toremifene is advantageously administered orally in a dosage of about 60 mg once a day, continuing the therapy for sufficient time to achieve and maintain a therapeutic effect. Anastrozole is advantageously administered orally in a dosage of about 1 mg once a day. Droloxifene is advantageously administered orally in a dosage of about 20-100 mg once a day. Raloxifene is advantageously administered orally in a dosage of about 60mg once a day. Exemestane is advantageously administered orally in a dosage of about 25 mg once a day. These dosages may be administered for example once, twice or more per course of treatment, which may be repeated for example every 7, 14, 21 or 28 days.

In view of their useful pharmacological properties, the components of the combinations according to the invention, i.e. the other medicinal agent and the HDAC inhibitor may be formulated into various pharmaceutical forms for administration purposes. The components may be formulated separately in individual pharmaceutical compositions or in a unitary pharmaceutical composition containing both components.

The present invention therefore also relates to a pharmaceutical composition comprising the other medicinal agent and the HDAC inhibitor together with one or more pharmaceutical carriers.

The present invention also relates to a combination according to the invention in the form of a pharmaceutical composition comprising an anti-cancer agent and a HDAC inhibitor according to the invention together with one or more pharmaceutical carriers.

The present invention further relates to the use of a combination according to the invention in the manufacture of a pharmaceutical composition for inhibiting the growth of tumour cells.

The present invention further relates to a product containing as first active ingredient a HDAC inhibitor according to the invention and as second active ingredient an anticancer agent, as a combined preparation for simultaneous, separate or sequential use in the treatment of patients suffering from cancer.

### Experimental part

The following examples illustrate the present invention.

Hereinafter, "DMF" is defined as *N*,*N*-dimethylformamide, "DCM" is defined as dichloromethane, "THF" is defined as tetrahydrofuran, "TFA" is defined as trifluoroacetic acid, "EtOAc" is defined as ethyl acetate, "EtOH" is defined as ethanol, "MeOH" is defined as methanol, "DIPE" is defined as diisopropyl ether, "EDC" is defined as *N*-(ethylcarbonimidoyl)-*N,N-*dimethyl-1,3-propanediamine, monohydrochloride and "HOBT" is defined as 1-hydroxy-1*H* benzotriazole.

### A. Preparation of the intermediate compounds

### Example A1

a) Preparation of intermediate1. 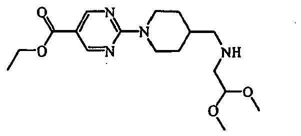 A mixture of 2-[4-(aminomethyl)-1-piperidinyl]- 5-pyrimidinecarboxylic acid, ethyl ester (0.0050 mol) and dimethoxy- acetaldehyde (0.0065 mol) in THF (30 ml) was stirred for one hour at 50°C. The mixture was cooled to room temperature. sodium triacetoxyborohydride (0.0065 mol) was added and the reaction mixture was shaken overnight at room temperature. DCM (20 ml) was added. The reaction was quenched with water (10 ml) and NaHCO₃. The layers were separated. The organic layer was dried, filtered and the solvent evaporated, yielding intermediate 1 (used in next reaction step, without further purification).
b) Preparation of intermediate 2 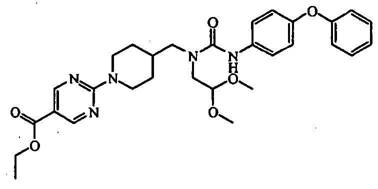 Intermediate 1 (0.0010 mol) and triethylamine (0.0020 mol) were dissolved in DCM (5 ml). 1-isocyanato-4-phenoxy- benzene (0.0015 mol) was added). The reaction mixture was shaken overnight at room temperature. Then, Tris-(2-aminoethyl)-amine polystyrene HL (Novabiochem; Cat. No. 01-64-0170) (0.0016 mol) was added. The reaction mixture was shaken for 3 hours at room temperature, then filtered and the filtrate was concentrated, yielding intermediate 2.
c) Preparation of intermediate 3 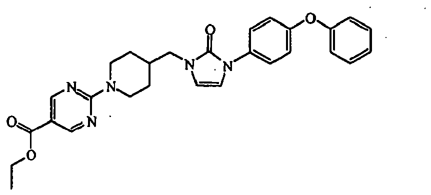 Formic acid (2 ml) was added to intermediate 2 (0.0010 mol). The reaction mixture was shaken for one hour at 50°C in a closed reaction vessel. The solvent was evaporated at 50°C in Genevac. The residue was taken up into DCM (15 ml), washed twice with 10% aqueous NaHCO₃ solution (2 ml), dried, then rinsed again with DCM. The solvent was evaporated, yielding intermediate 3 (used in next reaction step, without further purification).
d) Preparation of intermediate 4 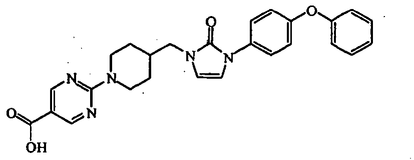 Intermediate 3 (0.0005 mol) was dissolved in THF (4 ml). Sodium hydroxide 1N (2 ml) was added and the reaction mixture was shaken overnight at room temperature in a closed vessel. Hydrochloric acid 1N (2 ml) was added, mixed, and then the solvent was evaporated, yielding intermediate 4 (used in next reaction step, without further purification).
e) Preparation of intermediate 5. 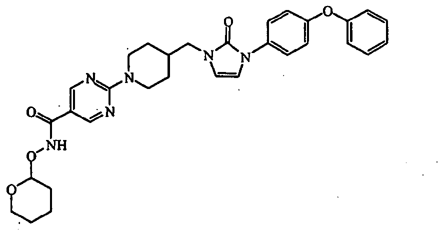
Intermediate 4 (max. 0.0005 mol), *N*'-(ethylcarbonimidoyl)-*N*,*N*-dimethyl-1,3-propanediamine, monohydrochloride (0.00065 mol), 1-hydroxy- 1*H*-benzotriazole (0.00065 mol) and triethylamine (0.00075 mol) were dissolved in THF (15 ml) at room temperature. Then, *O*-(tetrahydro-2*H*-pyran-2-yl)- hydroxylamine [6723-30-4] (0.00065 mol) was added and the reaction mixture was stirred for 6 hours at room temperature. The solvent was evaporated. DCM (25 ml) was added. The organic layer was separated, washed with a 10% aqueous NaHCO₃ solution (3 ml), then dried (filtered) through Extrelut ® cartridges and the solvent was evaporated. Each residue was purified by reversed-phase prep. HPLC. The product fractions were collected and the solvent was evaporated, yielding intermediate 5.

Following table lists the intermediates that were prepared according to one of the above Examples.

| | |
|---|---|
| 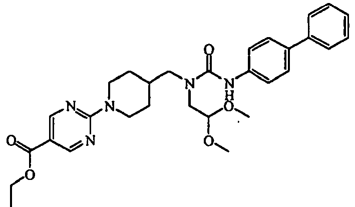 | 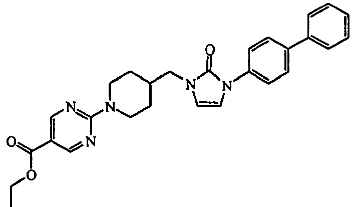 |
| Interm. No. 6; Ex. A1b | Interm. No. 7; Ex. A1c |
| 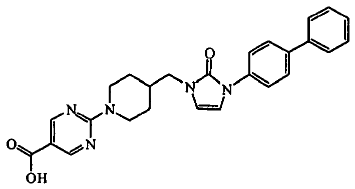 | 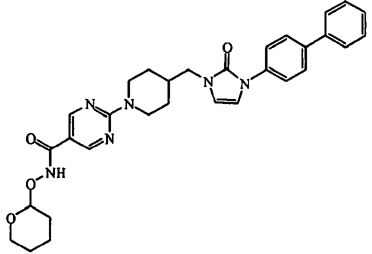 |
| Interm. No. 8; Ex. A1d | Interm. No. 9; Ex. A1e |

### Example A2

a) Preparation of intermediate 10 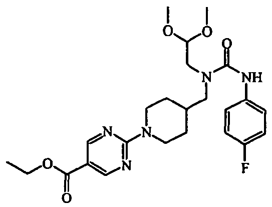 Intermediate 1 (0.0003 mol) was taken up into DCM (26 ml). 1-fluoro-4-isocyanatobenzene (0.0004 mol) was added. Then, triethylamine (0.0006 mol) was added. The reaction mixture was shaken overnight at room temperature. Tris-(2-aminoethyl)-amine polystyrene HL (Novabiochem Cat. No. 01-64-0170) (0.0004 mol) was added and the mixture was shaken for 3 hours at room temperature, then filtered and the filtrate was evaporated, yielding intermediate 10.
b) Preparation of intermediate 11 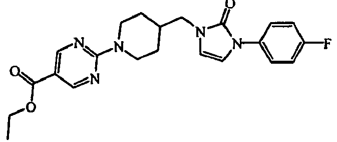 Formic acid (1 ml) was added to intermediate 10 (0.0003 mol). The reaction mixture was shaken for one hour at 50°C. The solvent was evaporated, yielding intermediate 11.
c) Preparation of intermediate 12 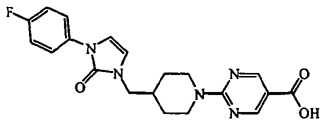 1N aqueous sodiumhydroxide/THF 1:1 (0.0010 mol) was added to intermediate 11 (1 0.0003 mol) and the mixture was shaken overnight at 50°C. 1N aqueous hydrochloric acid (0.0010 mol) was added and the solvent was evaporated, yielding intermediate 12.
d) Preparation of intermediate 13 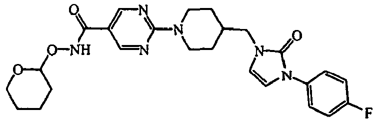 Intermediate 12 (< 0.0003 mol) was dissolved in DMF (1 ml). Then a solution of *N*'-(ethylcarbonimidoyl)-*N,N*-dimethyl-1,3-propanediamine, monohydrochloride (0.0004 mol), 1-hydroxy-1*H*-benzotriazole (0.0004 mol) and triethylamine (0.00045 mol) in THF (3 ml) and DCM (1 ml) was added. Then, a solution of *O*-(tetrahydro-2*H*-pyran-2-yl)- hydroxylamine (0.0004 mol) in DCM (1 ml) was added. The reaction mixture was shaken overnight at room temperature. DCM (20 ml) was added and the reaction mixture was washed with a 10% aqueous NaHCO₃ solution, then dried over Extrelut®. The filtrate was evaporated. The residue was purified by reversed-phase HPLC. The product fractions were collected and the solvent was evaporated, yielding intermediate 13.

Following table lists the intermediates that were prepared according to one of the above examples

| | |
|---|---|
| 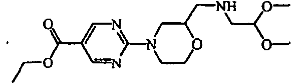 | |
| Interm. No.41, Ex A1a | |
| 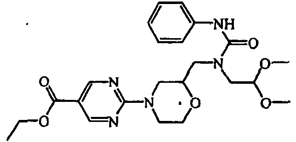 | 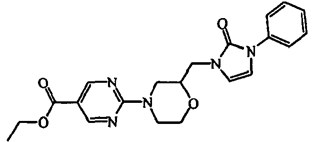 |
| Interm. No.28; Ex. A2a | Interm. No. 29; Ex. A2b |
| 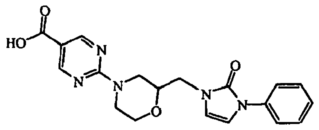 | 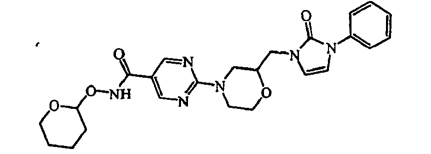 |
| Interm. No. 30; Ex. A2c | Interm. No. 31; Ex. A2d |

### Example A3

a) Preparation of intermediate 14 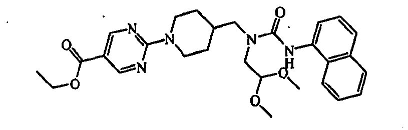 Intermediate 1 (0.0010 mol) and triethylamine (0.0020 mol) were dissolved in DCM (5 ml). 1-isocyanato- naphthalene (0.0015 mol) was added. Then, tris-(2-aminoethyl)-amine polystyrene HL (Novabiochem; Cat. No. 01-64-0170) (0.0016 mol) was added. The reaction mixture was shaken for 3 hours at room temperature, then filtered and the filtrate was evaporated, yielding intermediate 14.
b) Preparation of intermediate 15 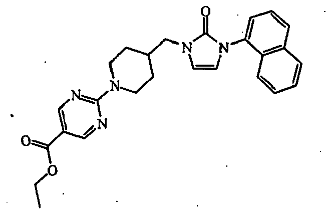 Formic acid (2 ml) was added to intermediate 14 (0.0010 mol). The reaction mixture was shaken for one hour at 50°C in a closed reaction vessel. The solvent was evaporated at 50°C. The residue was taken up into DCM (15 ml), washed twice with 10% aqueous NaHCO₃ solution (2 ml), then dried over Extrelut®, then rinsed again with DCM. The solvent was evaporated, yielding intermediate 15 (used in next reaction step, without further purification).
c) Preparation of intermediate 16 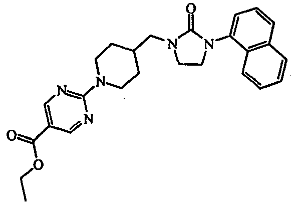 A mixture of intermediate 15 (0.0005 mol) and triethylamine (0.100 g) in THF (30 ml) was hydrogenated overnight at room temperature with Pd/C 10% (0.100 g) as a catalyst. After uptake of H₂ (1 equiv), the catalyst was filtered off over dicalite and the filtrate was evaporated, yielding intermediate 16.
d) Preparation of intermediate 17 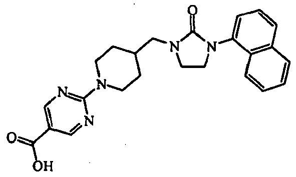 Intermediate 16 (0.0005 mol) was dissolved in 1 N aqueous sodium hydroxide solution (3 ml). THF (3 ml) was added. The reaction mixture was shaken overnight at room temperature in a closed vessel. 1N aqueous hydrochloric acid solution (3 ml) was added and the solvent was evaporated, yielding intermediate 17 (used in next reaction step, without further purification).
e) Preparation of intermediate 18 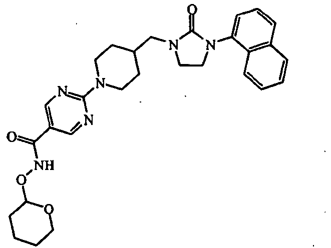 Intermediate 17 (0.0005 mol) and *N'*-(ethylcarbonimidoyl)-*N,N*-dimethyl-1,3-propanediamine, monohydrochloride (0.00065 mol), 1-hydroxy-1*H* benzotriazole (0.00065 mol) and triethylamine (0.00075 mol) were dissolved in THF (15 ml) at room temperature. Then, O-(tetrahydro-2*H*-pyran-2-yl)- hydroxylamine (0.00065 mol) was added. The reaction mixture was stirred for 6 hours at room temperature. The solvent was evaporated. DCM (25 ml) was added to the residue and the organic solution was washed with a 10% aqueous NaHCO₃ solution (3 ml), then dried through Extrelut® cartridges. The solvent was evaporated. The residue was purified by prep. reversed-phase HPLC. The product fractions were collected and the solvent was evaporated, yielding 0.026 g of intermediate 18.

### Example A4

a) Preparation of intermediate 19 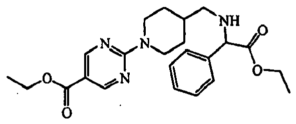 A mixture of 2-[4-(aminomethyl)-1-piperidinyl]- 5-pyrimidinecarboxylic acid, ethyl ester (0.0189 mol), α-chloro- benzeneacetic acid, ethyl ester (0.0199 mol) and potassium carbonate (5.2g) in acetonitrile (125ml) was stirred and refluxed for 15 hours, poured out into water and extracted with EtOAc. The organic layer was separated, dried (MgSO₄), filtered, and the solvent was evaporated. The residue (8.5g) was purified by column chromatography over silica gel (15-40µm) (eluent: DCM/EtOAc 95/5 to 90/10). The pure fractions were collected and the solvent was evaporated, yielding 3.5g (43%) of intermediate 19.
b) Preparation of intermediate 20 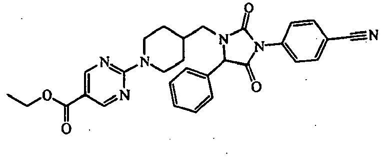 4-isocyanato- benzonitrile (0.0021 mol) was added to a mixture of intermediate 19 (0.0014 mol) in THF (25ml). The mixture was stirred at room temperature for 24 hours, poured out into water and extracted with EtOAc. The organic layer was separated, dried (MgSO₄), filtered, and the solvent was evaporated. The residue (1g) was purified by column chromatography over silica gel (5µm) (eluent: DCM/MeOH 100/0 to 94/6). The pure fractions were collected and the solvent was evaporated. The residue-(0.47g) was taken up in EtOAc and dried, yielding 0.36 g of intermediate 20, melting point 204°C.
c) Preparation of intermediate 21 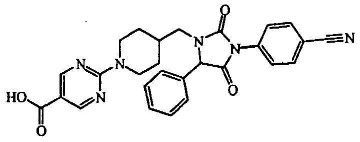 A mixture of intermediate 20 (0.0007 mol) in hydrochloric acid 3N (15ml) and dioxane (15ml) was stirred and refluxed for 8 hours. The solvent was evaporated. Water (30ml) was added. The precipitate was filtered, washed with water, then with DIPE and dried, yielding 0.34g (94%) of intermediate 21.
d) Preparation of intermediate 22 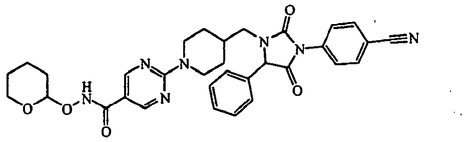 1-hydroxy-1*H-*benzotriazole (0.001 mol) then *N'*-(ethylcarbonimidoyl)-*N,N*-dimethyl-1,3-propanediamine, monohydrochloride (0.001 mol) were added to a solution of intermediate 21 (0.0007 mol), *O*-(tetrahydro-2*H*-pyran-2-yl)- hydroxylamine (0.001 mol) and triethylamine (0.002 mol) in DCM/THF (35ml) under N₂ flow. The mixture was stirred at room temperature for 3 days, poured out into water and extracted with DCM. The organic layer was separated, dried (MgSO₄), filtered, and the solvent was evaporated. The residue (0.5)g was purified by column chromatography over kromasil® (5µm) (eluent: DCM/MeOH 100/0 to 95/5). The pure fractions were collected and the solvent was evaporated. The residue (0.3 g) was crystallized from DIPE/diethyl ether. The precipitate was filtered off and dried. The residue (0.25g) was dried at 70°C for 4 hours, yielding 0.22g (54%) of intermediate 22, melting point 182°C.

### Example A5

a) Preparation of intermediate 23. 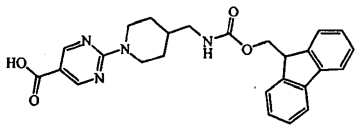 A mixture of 2-[4-(aminomethyl)-1-piperidinyl]-5-pyrimidinecarboxylic acid, ethyl ester (0.0072 mol) in THF (40ml) and sodium hydroxide 1N (40ml) was stirred at room temperature overnight. Hydrochloric acid 1N (40ml) was added. The mixture was stirred for 10 minutes. Sodium carbonate (0.0216 mol) was added. The mixture was stirred for 10 minutes. 1-[[(9*H*-fluoren-9-ylmethoxy)carbonyl]oxy]- 2,5-pyrrolidinedione (0.0072 mol) was added portionwise. The mixture was stirred at room temperature for 6 hours, then cooled to 0°C and acidified with hydrochloric acid. The precipitate was filtered, washed with diethyl ether and dried, yielding 4.1g (100%) of intermediate 23.
b) preparation of intermediate 24 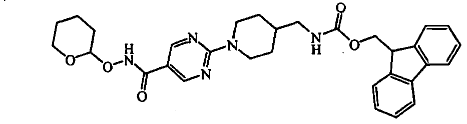 Triethylamine (0.02 mol), *N'*-(ethylcarbonimidoyl)-*N,N*-dimethyl-1,3-propanediamine, monohydrochloride (0.0082 mol) and 1-hydroxy-1*H* benzotriazole (0.0082 mol) were added at room temperature to a mixture of intermediate 23 (0.0068 mol) in DCM/THF (200ml) under N₂ flow. The mixture was stirred at room temperature for 15 minutes. *O-*(tetrahydro-2*H*-pyran-2-yl)-hydroxylamine (0.0082 mol) was added. The mixture was stirred at room temperature for 48 hours, poured out into water and extracted with DCM. The organic layer was washed with NaHCO₃ 10%, dried (MgSO₄), filtered, and the solvent was evaporated. The residue (4g) was purified by column chromatography over silica gel (15-40µm) (eluent: DCM/MeOH/NH₄OH 98/2/0.1). The pure fractions were collected and the solvent was evaporated, yielding 3.4g (89%) of intermediate 24.
c) Preparation of intermediate 25 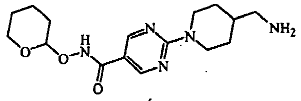 A mixture of intermediate 24 (0.0355 mol) and piperidine (0.089 mol) in DCM (400ml) was stirred at 35°C during 75 hours. The solvent was evaporated. The residue was purified by column chromatography over silica gel (15-40µm) (eluent: DCM/MeOH/NH₄OH 80/20/2). The pure fractions were collected and the solvent was evaporated, yielding 6.7g (56%). A fraction (0.79g) was crystallized from diethyl ether. The precipitate was filtered off and dried, yielding 0.62g of intermediate 25, melting point: 129°C.
d) Preparation of intermediate 26 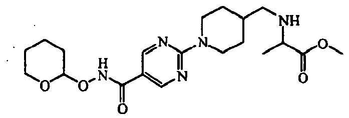 A mixture of intermediate 25 (0.0053 mol), 2-bromo- propanoic acid, methyl ester (0.0056 mol) and potassium carbonate (0.01 mol) in acetonitrile (75ml) was stirred and refluxed for 3 hours, then poured out into water and extracted with DCM. The organic layer was separated, dried (MgSO₄), filtered and the solvent was evaporated. The residue (2.2g) was purified by column chromatography over silica gel (15-40µm) (eluent: DCM/MeOH/NH₄OH 97/3/0.1 to 96/4/0.5). The pure fractions were collected and the solvent was evaporated, yielding 0.62g (28%) of intermediate 26.
e) Preparation of intermediate 27 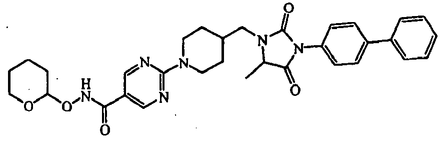 4-isocyanato- 1,1'-biphenyl (0.0009 mol) was added at room temperature to a solution of intermediate 26 (0.0006 mol) in THF (14ml). The mixture was stirred at room temperature for 72 hours, poured out into water and extracted with EtOAc. The organic layer was separated, dried (MgSO₄), filtered, and the solvent was evaporated. This fraction (0.75g) was purified by column chromatography over silica gel (5µm) (eluent: DCM/MeOH 100/0 to 90/10). The pure fractions were collected and the solvent was evaporated, yielding 0.15g (43%) of intermediate 27.

### Example A6

a) Preparation of intermediate 32 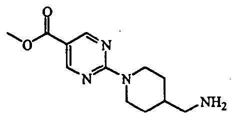 A solution of 2-chloro-5-pyrimidinecarboxylic acid, methyl ester (0.058 mol) in *N,N* dimethyl- acetamide (80ml) was added dropwise to a solution of 4-piperidinemethanamine (0.116 mol) and *N*-ethyl-*N*-(1-methylethyl)- 2-propanamine (0.145 mol) in *N*,*N*-dimethyl- acetamide (150ml) under N₂ flow. The mixture was stirred at room temperature for 1 hour and 30 minutes, poured out into ice water and extracted with EtOAc, then with DCM. The organic layer was washed with water, dried (MgSO₄), filtered and the solvent was evaporated. The residue was crystallized from DIPE. The precipitate was filtered off and dried, yielding 10g (65%) of intermediate 32.
b) Preparation of intermediate 33 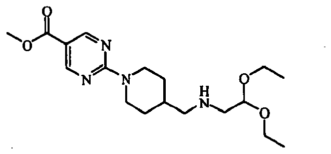 A mixture of intermediate 32 (0.012 mol) and potassium carbonate (0.0383 mol) in acetonitrile (50ml) was stirred at reflux for 2 hours. 2-bromo-1,1-diethoxy- ethane (0.0479 mol) was added. The mixture was stirred and refluxed for 15 hours, poured out into water and extracted with DCM. The organic layer was separated, dried (MgSO₄), filtered and the solvent was evaporated. The residue (8g) was purified by column chromatography over silica gel (15-40µm) (eluent: DCM/MeOH/NH₄OH 98/2/0.1). The pure fractions were collected and the solvent was evaporated, yielding 1.9g (43%) of intermediate 33.
c) Preparation of intermediate 34 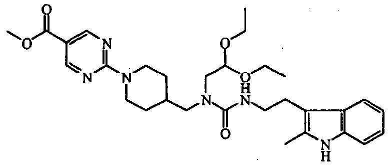 A solution of intermediate 33 (0.0022 mol) and triethylamine (0.003 mol) in THF (25ml) was added dropwise at 5°C to a solution of trichloro- methanol, carbonate(2:1) (0.0009 mol) in THF (15ml). The mixture was stirred at room temperature for 2 hours. A solution of 2-methyl-1*H-*indole-3-ethanamine (0.0026 mol) and triethylamine (0.003 mol) in THF (25ml) was added dropwise. The mixture was stirred at 50°C for 15 hours, poured out into ice water and extracted twice with EtOAc. The organic layer was washed with saturated NaCl, dried (MgSO₄), filtered and the solvent was evaporated. The residue (1.6 g) was purified by column chromatography over silica gel (5 µm) (eluent: DCM/MeOH/NH₄OH 99/1/0.05 to 94/6/0.3). The pure fractions were collected and the solvent was evaporated, yielding 0.73g of intermediate 34.
d) Preparation of intermediate 35 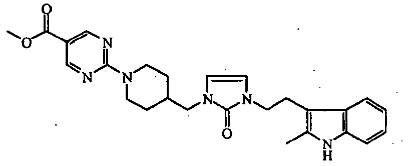 A mixture of intermediate 34 (0.0013 mol) in hydrochloric acid 1N (20ml) and MeOH (20ml) was stirred at 55°C for 3 hours. Methanol was evaporated. The residue was basified with potassium carbonate. The mixture was extracted with EtOAc. The organic layer was washed with saturated NaCl, dried (MgSO₄), filtered and the solvent was evaporated. The residue (0.64g) was purified by column chromatography over silica gel (15-40µm) (eluent: DCM/MeOH/NH₄OH 97/3/0.1). The pure fractions were collected and the solvent was evaporated, yielding 0.52g (87%) of intermediate 35, melting point 85°C.
e) Preparation of intermediate 36 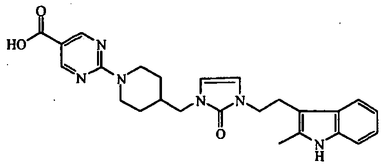 A mixture of intermediate 35 (0.0011 mol) and lithium hydroxide (0.022 mol) in THF (20ml) and water (10ml) was stirred at room temperature for 15 hours, then taken up in hydrochloric acid 3N till pH was set to 4. The mixture was extracted with DCM. The organic layer was separated, dried (MgSO₄), filtered, and the solvent was evaporated, yielding 0.5g (100%) of intermediate 36.
f) Preparation of intermediate 37 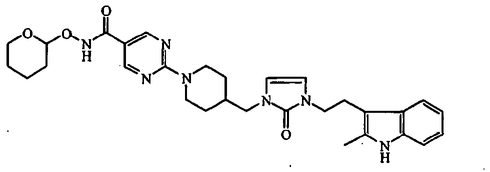 *N*-(ethylcarbonimidoyl)-*N*,*N*-dimethyl-1,3-propanediamine, monohydrochloride (0.0016 mol) and 1-hydroxy-1*H*-benzotriazole (0.0016 mol) were added at room temperature to a solution of intermediate 36 (0.001 mol), *O*-(tetrahydro-2*H*-pyran-2-yl)- hydroxylamine (0.0016 mol) and triethylamine (0.0032 mol) in DCM/THF (50/50) (40ml). The mixture was stirred at room temperature for 48 hours, poured out into water and extracted with DCM. The organic layer was separated, dried (MgSO₄), filtered and the solvent was evaporated. The residue (0.74g) was purified by column chromatography over silica gel (10µm) (eluent: DCM/MeOH/NH₄OH 97/3/0.1). The pure fractions were collected and the solvent was evaporated, yielding 0.49g (80%) of intermediate 37, melting point 116°C.

### Example A7

a) Preparation of intermediate 38 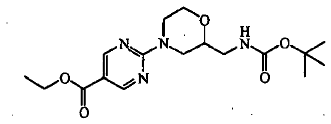 A solution of 2-(methylsulfonyl)- 5-pyrimidinecarboxylic acid, ethyl ester (0.0118 mol) in acetonitrile (30ml) was added dropwise to a solution of (2-morpholinylmethyl)-carbamic acid, 1,1-dimethylethyl ester (0.0098 mol) and potassium carbonate (0.0196 mol) in acetonitrile (80ml) under N₂ flow. The mixture was stirred at room temperature for 12 hours, poured out into water and extracted with EtOAc. The organic layer was separated, dried (MgSO₄), filtered, and the solvent was evaporated till dryness. The residue (5.6g) was purified by column chromatography over silica gel (15-35µm) (eluent: DCM/MeOH 99/1). The pure fractions were collected and the solvent was evaporated. The residue was crystallized from diethyl ether. The precipitate was filtered off and dried, yielding 0.3g of intermediate 38, melting point 100°C.
b) Preparation of intermediate 39 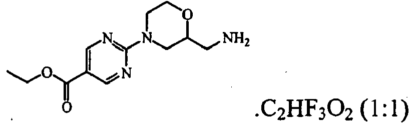 TFA (7.5ml) was added at 0°C to a mixture of intermediate 38 (0.037 mol) in DCM (150ml). The mixture was stirred at room temperature for 48 hours. The solvent was evaporated. Diethyl ether was added. The precipitate was filtered off and dried, yielding 13.5g (96%) of intermediate 39, melting point 180°C.
c) Preparation of intermediate 40 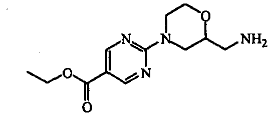 free base Intermediate 39 (0.0105 mol) was added to a solution of potassium carbonate 10% (100ml) in DCM (100ml). The mixture was stirred at room temperature for 15 minutes, then extracted with DCM. The organic layer was separated, dried (MgSO₄), filtered and the solvent was evaporated, yielding 2.6g of intermediate 40.

### Example A8

a) Preparation of intermediate 42 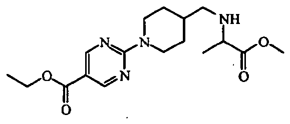 A mixture of 2-[4-(aminomethyl)-1-piperidinyl]- 5-pyrimidinecarboxylic acid, ethyl ester (0.0007 mol), 2-bromo- propanoic acid, methyl ester (0.0007 mol) and potassium carbonate (0.0015 mol) in acetonitrile (5ml) was stirred and refluxed for 3 hours, poured out into water and extracted with DCM. The organic layer was separated, dried (MgSO₄), filtered, and the solvent was evaporated, yielding 0.24g of intermediate 42.
b) Preparation of intermediate 43 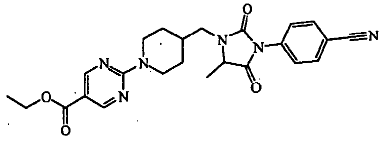 4-isocyanato- benzonitrile (0.0006 mol) was added at room temperature to a solution of intermediate 42 (0.0006 mol) in THF (15ml). The mixture was stirred and refluxed for 15 hours. 4-isocyanato- benzonitrile (1eq) was added. The mixture was stirred and refluxed for 24 hours, poured out into water and extracted with DCM. The organic layer was separated, dried (MgSO₄), filtered, and the solvent was evaporated. The residue was taken up in a minimum of DCM. The precipitate was eliminated by filtration. The filtrate was evaporated and purified by column chromatography over silica gel (20µm) (eluent: DCM/MeOH/NH₄OH 98/2/0.1). The pure fractions were collected and the solvent was evaporated, yielding 0.16g (48%) of intermediate 43.
c) Preparation of intermediate 44 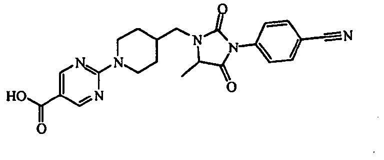 A mixture of intermediate 43 (0.0003 mol) and lithium hydroxide monohydrate (0.0007 mol) in THF (5ml) and water (2ml) was stirred at room temperature for 15 hours. Hydrochloric acid 1N was added. The mixture was evaporated till dryness. This product was used directly in the next reaction step, yielding intermediate 44.
d) Preparation of intermediate 45 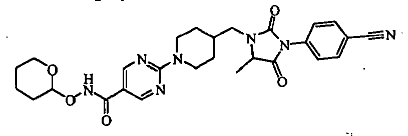 A mixture of intermediate 44 (0.0003 mol), O-(tetrahydro-2*H*-pyran-2-yl)-hydroxylamine (0.0004 mol), EDC (0.0004 mol), HOBT (0.0004 mol) and triethylamine (0.0005 mol) in DCM/THF (20ml) was stirred at room temperature for 48 hours, poured out into water and extracted with DCM. The organic layer was separated, dried (MgSO₄), filtered, and the solvent was evaporated. The residue (0.2g) was purified by column chromatography over kromasil® (10µm) (eluent: DCM/MeOH 99/1). The pure fractions were collected and the solvent was evaporated, yielding 0.108g (59%) of intermediate 45, melting point 120°C.

### B. Preparation of the final compounds

### Example B1

### Preparation of compound 1

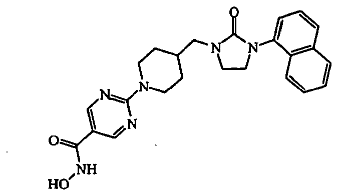

TFA 5% in DCM/MeOH (2 ml) was added to intermediate 18 (0.00005 mol) and the resultant solution was shaken in sealed tubes at room temperature overnight. The solvent was evaporated under a N₂ flow at room temperature. TFA 5% in DCM/MeOH was added again and the mixture was shaken until deprotection was complete (checked by LC/MS). The solvent was evaporated under N₂ flow at room temperature. 1,4-Dioxane (2 ml) was added, and the evaporation procedure was repeated at 40°C to give the final product, yielding 0.022 g of compound 1.

### Example B2

### Preparation of compound 2

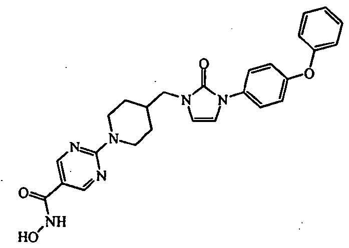

5 % TFA in DCM/MeOH 1/1 (4 ml) was added to intermediate 5 (max. 0.0005 mol) and the resultant solution was shaken in sealed tubes at room temperature overnight. The solvent was evaporated under a N₂ flow at room temperature. 5 % TFA in DCM/MeOH 1/1 (4 ml) was added again and the mixture was shaken until deprotection was complete (checked by LC/MS). The solvent was evaporated under N₂ flow at room temperature. 1,4-Dioxane (2 ml) was added, and the evaporation procedure was repeated at 40°C to give the final products, yielding compound 2.

### Example B3

### Preparation of compound 3

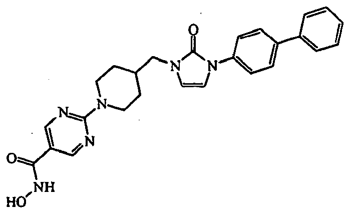

5 % TFA in DCM/MeOH 1/1 (4 ml) was added to intermediate 9 (max. 0.0005 mol) and the resultant solution was shaken in sealed tubes at room temperature overnight. The solvent was evaporated under a N₂ flow at room temperature. 5 % TFA in DCM/MeOH 1/1 (4 ml) was added again and the mixture was shaken until deprotection was complete (checked by LC/MS). The solvent was evaporated under N₂ flow at room temperature. 1,4-Dioxane (2 ml) was added, and the evaporation procedure was repeated at 40°C to give compound 3.

### Example B4

### Preparation of compound 4

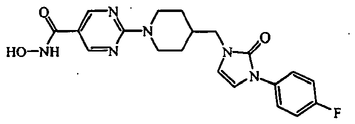

5% TFA in DCM/MeOH (2 ml) was added to intermediate 13 (0.0003 mol) and the resultant solution was left to stand for 3 days at room temperature. The solvent was evaporated under N₂ flow and this procedure was repeated until the product was pure, yielding compound 4.

### Example B5

### Preparation of compound 5

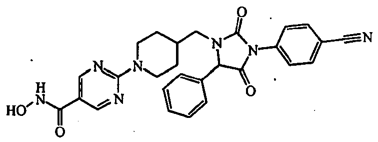

A mixture of intermediate 22 (0.0003 mol) in TFA (1ml), MeOH (20ml) and DCM (5ml) was stirred at room temperature for 96 hours. DCM was evaporated. The precipitate was filtered, washed with a minimum of MeOH, then with diethyl ether and dried, yielding 0.135g (83%) of compound 5, melting point 187°C.

### Example B6

### Preparation of compound 6

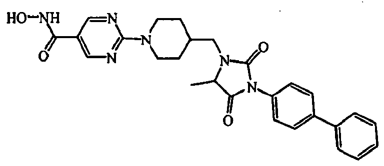

TFA (0.5ml) was added at 5°C to a solution of intermediate 27 (0.0002 mol) in MeOH (10ml). The mixture was stirred at room temperature for 24 hours. The solvent was evaporated till dryness. The residue was purified by column chromatography over silica gel (25-40µm) (eluent: DCM/MeOH/water 80/20/2;). The pure fractions were collected and the solvent was evaporated. The residue (0.09g) was taken up in diethyl ether. The precipitate was filtered off and dried, yielding 0.072g (56%) of compound 6, melting point: 169°C.

### Example B7

### Preparation of compound. 12

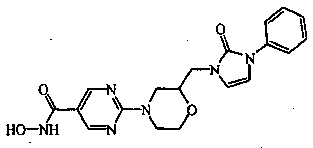

5% TFA in DCM/MeOH (2 ml) was added to intermediate 31 (0.0003 mol) and the resultant solution was left to stand for 3 days at room temperature. The solvent was evaporated under N₂ flow and this procedure was repeated until the product was pure, yielding compound 12.

### example B8

### Preparation of compound 35

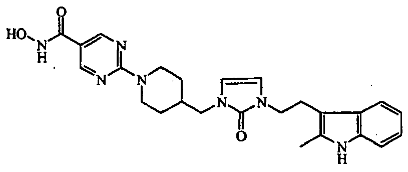

A mixture of intermediate 37 (0.0008 mol) in TFA (2.2ml) and MeOH (44ml) was stirred at room temperature for 24 hours. The solvent was evaporated. The precipitate was filtered, washed with MeOH, then with diethyl ether and dried, yielding 0.31g (84%) of compound 35, melting point 246°C.

### Example B9

### Preparation of compound 36

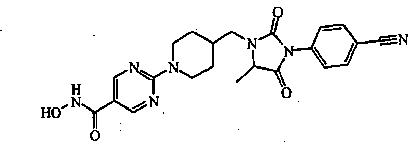

A mixture of intermediate 45 (0.0002 mol) in TFA (0.5ml) and MeOH (10ml) was stirred at room temperature for 24 hours and the solvent was evaporated till dryness. The residue was crystallized from diethyl ether/2-propanone. The precipitate was filtered off and dried, yielding 0.05g (66%) of compound 36, melting point 135°C.

**Table F-1**

| | |
|---|---|
| 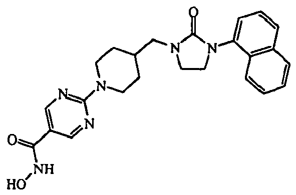 | 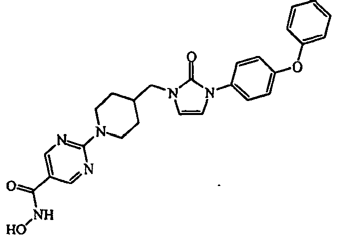 |
| Co. No. 1; Ex. [B1]; Rt: 3.86, (MH)⁺: 447, Method 1 | Co. No. 2; Ex. [B2]; Rt: 4.25, (MH)⁺: 487, Method 1 |
| 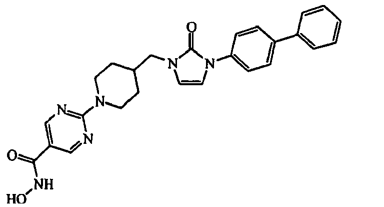 | 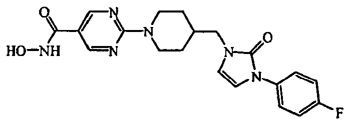 |
| Co. No. 3; Ex. [B3]; Rt: 4.3, (MH)⁺: 471, Method 1 | Co. No. 4; Ex. [B4]; Rt: 5.61, (MH)⁺: 413, Method 2 |
| 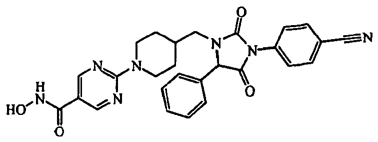 | 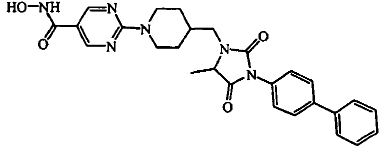 |
| Co. No. 5; Ex. [B5]; mp. 187 °C; Rt: 7.93, (MH)⁺: 512, Method 3 | Co. No. 6; Ex. [B6]; mp. 169 °C; Rt: 8.23, (MH)⁺: 501, Method 3 |
| 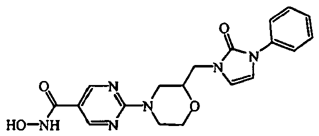 | 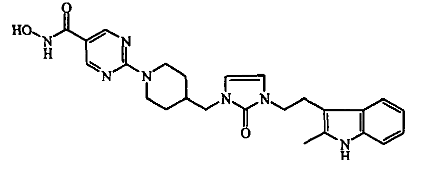 |
| Co. No. 12; Ex. [B7]; Rt: 4.68; (MH)⁺: 397, Method 1 | Co. No. 35; Ex. [B8]; mp. 246 °C; Rt: 7.84, (MH)⁺: 476, Method 4 |
| 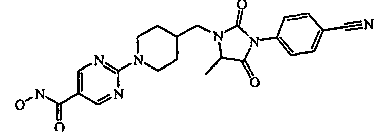 | |
| Co. No. 36; Ex. [B9]; mp. 135 °C; Rt: 7.16, (MH)⁺: 450, Method 3 | |
| 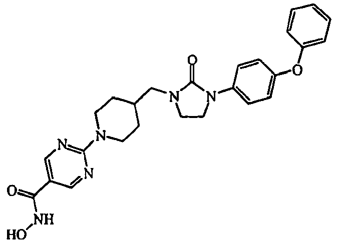 | 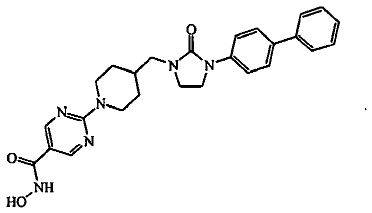 |
| Co. No. 7; Ex. [B1]; Rt: 4.28, (MH)⁺: 489, Method 1 | Co. No. 8; Ex. [B1]; Rt: 4.44, (MH)⁺: 473, Method 1 |
| 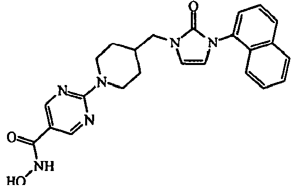 | 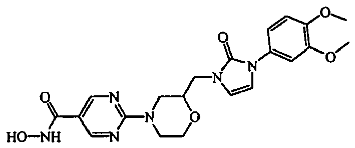 |
| Co. No. 9; Ex. [B2, B3]; Rt: 3.86, (MH)⁺: 445, Method 1 | Co. No. 10; Ex. [B4]; Rt: 4.78, (MH)⁺: 457, Method 1 |
| 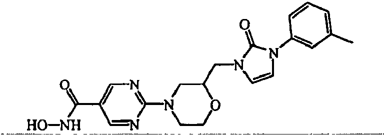 | |
| Co. No. 11; Ex. [B4]; Rt: 5.69, (MH)⁺: 411, Method 1 | |
| 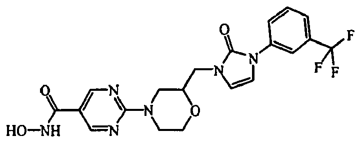 | 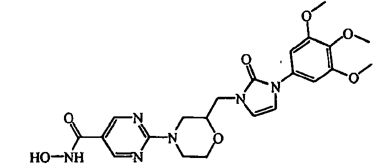 |
| Co. No. 13; Ex. [B7]; Rt: 6.41, (MH)⁺: 465, Method 2 | Co. No. 14; Ex. [B7]; Rt: 4.99, (MH)⁺: 487, Method 2 |
| 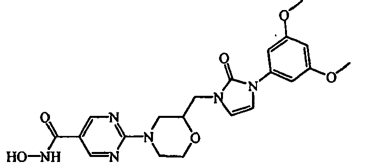 | 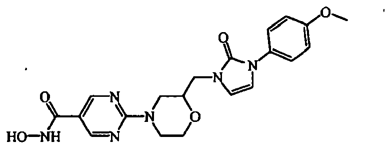 |
| Co. No. 15; Ex. [B7]; Rt: 5.7, (MH)⁺: 457, Method 2 | Co. No. 16; Ex. [B7]; Rt: 5.13, (MH)⁺: 427, Method 2 |
| 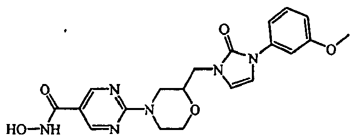 | 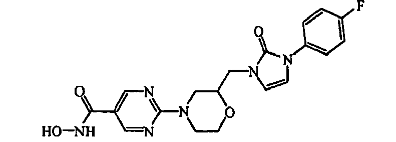 |
| Co. No. 17; Ex. [B7]; Rt: 5.32, (MH)⁺: 427, Method 2 | Co. No. 18; Ex. [B7]; Rt: 5.2, (MH)⁺: 415, Method 2 |
| 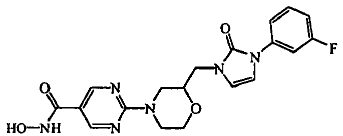 | 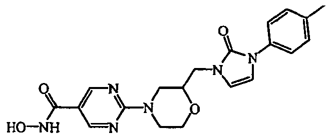 |
| Co. No. 19; Ex. [B7]; Rt: 5.41, (MH)⁺: 415, Method 2 | Co. No. 20; Ex. [B7]; Rt: 5.64, (MH)⁺: 411, Method 2 |
| 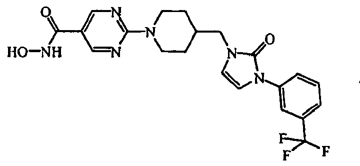 | 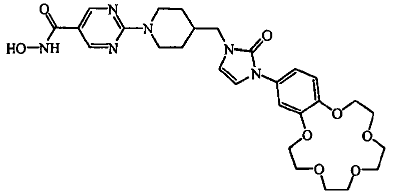 |
| Co. No. 21; Ex. [B4]; Rt: 6.74, (MH)⁺: 463, Method 2 | Co. No. 22; Ex. [B4]; Rt: 4.89, (MH)⁺: 585, Method 2 |
| 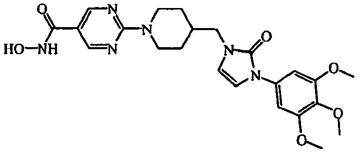 | 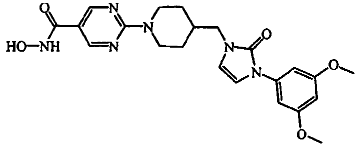 |
| Co. No. 23; Ex. [B4]; Rt: 5.29, (MH)⁺: 485, Method 2 | Co. No. 24; Ex. [B4]; Rt: 6.02, (MH)⁺: 455, Method 2 |
| 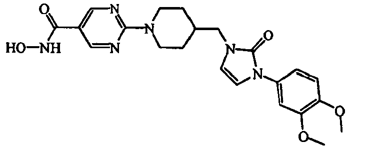 | 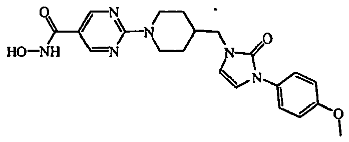 |
| Co. No. 25; Ex. [B4]; Rt: 5.1, (MH)⁺: 455, Method 2 | Co. No. 26; Ex. [B4]; Rt: 5.49, (MH)⁺: 425, Method 2 |
| 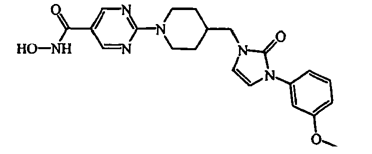 | 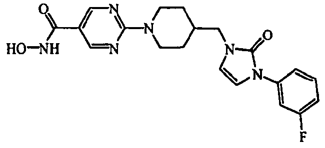 |
| Co. No. 27; Ex. [B4]; Rt: 5.7, (MH)⁺: 425, Method 2 | Co. No. 28; Ex. [B4]; Rt: 5.86, (MH)⁺: 413, Method 2 |
| 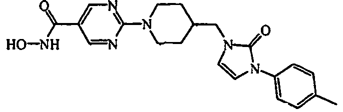 | 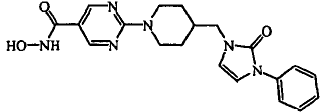 |
| Co. No. 29; Ex. [B4]; Rt: 6.03, (MH)⁺: 409, Method 2 | Co. No. 30; Ex. [B4]; Rt: 5.41, (MH)⁺: 395, Method 2 |
| 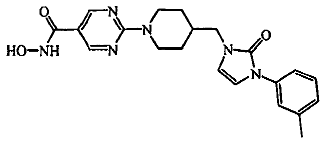 | 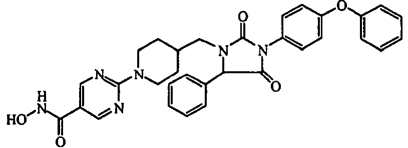 |
| Co. No. 31; Ex. [B4]; Rt: 6.04, (MH)⁺: 409, Method 2 | Co. No. 32; Ex. [B5]; mp. 154°C; Rt: 8.9, (MH)⁺: 579, Method 3 |
| 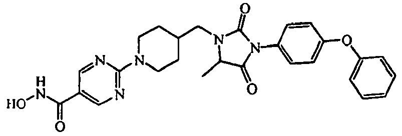 | 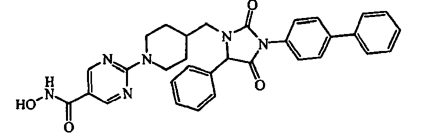 |
| Co. No. 33; Ex. [B6]; mp. 149°C; Rt: 8.24, (MH)⁺: 517, Method 3 | Co. No. 34; Ex. [B6]; mp. 158°C; Rt: 8.87, (MH)⁺: 563, Method 3 |

### General HPLC procedure A

The HPLC gradient was supplied by an Alliance HT 2790 (Waters) system consisting of a quaternary pump with degasser, an autosampler, a column oven (set at 40 °C) and DAD detector. Flow from the column was split to the MS detector. MS detectors were configured with an electrospray ionization source. Mass spectra were acquired by scanning from 100 to 1000 in 1 second using a dwell time of 0.1 second. The capillary needle voltage was 3 kV and the source temperature was maintained at 140 °C. Nitrogen was used as the nebulizer gas. Data acquisition was performed with a Waters-Micromass MassLynx-Openlynx data system.

### General HPLC procedure B

The HPLC gradient was supplied by an Alliance HT 2795 (Waters) system consisting of a quaternary pump with degasser, an autosampler, and DAD detector. Flow from the column was split to the MS detector. MS detectors were configured with an electrospray ionization source. The capillary needle voltage was 3 kV and the source temperature was maintained at 100 °C. Nitrogen was used as the nebulizer gas. Data acquisition was performed with a Waters-Micromass MassLynx-Openlynx data system. *Method 1* In addition to general procedure A: Reversed phase HPLC was carried out on an Xterra MS C18 column (3.5 mm, 4.6 x 100 mm) with a flow rate of 1.6 ml/min. Three mobile phases (mobile phase A: 95% 25 mM ammoniumacetate + 5 % acetonitrile; mobile phase B: acetonitrile; mobile phase C: methanol) were employed to run a gradient condition from 100 % A to 50 % B and 50 % C in 6.5 minutes, to 100 % B in 1 minute, 100 % B for 1 minute and reequilibrate with 100 % A for 1.5 minutes. An injection volume of 10 µl was used.
Cone voltage was 10 V for positive ionization mode and 20 V for negative ionization mode.

*Method* 2In addition to general procedure A: Reversed phase HPLC was carried out on an Xterra MS C18 column (3.5 mm, 4.6 x 100 mm) with a flow rate of 1.2 ml/min. Three mobile phases (mobile phase A: 95% 25 mM ammoniumacetate + 5 % acetonitrile; mobile phase B: acetonitrile; mobile phase C: methanol) were employed to run a gradient condition from 100 % A to 50 % B and 50 % C in 10 minutes, to 100 % B in 1 minute, 100 % B for 3 minutes and reequilibrate with 100 % A for 1.5 minutes. An injection volume of 10 µl was used.

*Method 3*In addition to general procedure B: Reversed phase HPLC was carried out on an Xterra-RP C 18 column (5 µm, 3.9 x 150 mm) with a flow rate of 1.0 ml/min. Two mobile phases (mobile phase A: 100 % 7 mM ammonium acetate; mobile phase B: 100 % acetonitrile; were employed to run a gradient condition from 85 % A, 15 % B (hold for 3 minutes) to 20 % A, 80 % B in 5 minutes, hold at 20 % A and 80 % B for 6 minutes and reequilibrate with initial conditions for 3 minutes. An injection volume of 20 µl was used.

Cone voltage was 20 V for positive ionization mode. Mass spectra were acquired by scanning from 100 to 900 in 0.8 seconds using an interscan delay of 0.08 seconds.

*Method 4*In addition to general procedure B: Identical to method 3, except that the ionization is both positive and negative. Cone voltage was 20 V for both positive and negative ionization mode.

### C. Pharmacological example:

The *in vitro* assay for inhibition of histone deacetylase (see example C.1) measures the inhibition of HDAC enzymatic activity obtained with the compounds of formula (I).

Cellular activity of the compounds of formula (I) was determined on A2780 tumour cells using a colorimetric assay for cell toxicity or survival (Mosmann Tim, Journal of Immunological Methods 65: 55-63, 1983)(see example C.2).

The solubility of a compound measures the ability of a compound to stay in solution. The solubility of a compound at different pH's can be measured with the use of a chemiluminescent nitrogen detector (see example C.3).

It has been shown that a wide variety of anti-tumoral agents activate the p21 protein, including DNA damaging agents and histone deacetylase inhibitors. DNA damaging agents activate the p21 gene through the tumour suppressor p53, while histone deacetylase inhibitors transcriptionally activates the p21 gene via the transcription factor Spl. Thus, DNA damaging agents activate the p21 promoter through the p53 responsive element while histone deacetylase inhibitors activate the p21 promoter through sp 1 sites (located at the -60 bp to +40 bp region relative to the TATA box) both leading to increased expression of the p21 protein. When the p21 promoter in a cells consists of a p21 1300 bp promoter fragment that does not comprise the p53 responsive elements it is accordingly non-responsive to DNA damaging agents. The capacity of compounds to induce p21 can be evaluated in several ways. A first method is to treat tumour cells with the compound of interest and after lysis of the cells detects p21 induction with the p21 enzyme linked immunosorbent assay (WAF1 ELISA of Oncogene). The p21 assay is a "sandwich" enzyme immunoassay employing both mouse monoclonal and rabbit polyclonal antibodies. A rabbit polyclonal antibody, specific for the human p21 protein, has been immobilized onto the surface of the plastic wells provided in the kit. Any p21 present in the sample to be assayed will bind to the capture antibody. The biotinylated detector monoclonal antibody also recognizes human p21 protein, and will bind to any p21, which has been retained by the capture antibody. The detector antibody, in turn, is bound by horseradish peroxidas-conjugated streptavidin. The horseradish peroxidase catalyses the conversion of the chromogenic substrate tetra-methylbenzidine from a colorless solution to a blue solution (or yellow after the addition of stopping reagent), the intensity of which is proportional to the amount of p21 protein bound to the plate. The colored reaction product is quantified using a spectrophotometer. Quantitation is achieved by the construction of a standard curve using known concentrations of p21 (provided lyophilised). This assay can measures p21 induction as the consequence of DNA damage or as the consequence of histone deacetylase inhibition (see example C.4.a.).

Another method tests the capacity of compounds to induce p21 as the consequence of HDAC inhibition at the cellular level. The cells can be stably transfected with an expression vector containing a p21 1300bp promoter fragment that does not comprise the p53 responsive elements and wherein an increase of a reporter gene expression, compared to the control levels, identifies the compound as having p21 induction capacity. The reporter gene is a fluorescent protein and the expression of the reporter gene is measured as the amount of fluorescent light emitted (see example C.4.b.).

Specific HDAC inhibitors should not inhibit other enzymes like the abundant CYP P450 proteins. The CYP P450 (E.coli expressed) proteins 3A4, 2D6 en 2C9 convert their specific substrates into a fluorescent molecule. The CYP3A4 protein converts 7-benzyloxy-trifluoromethyl coumarin (BFC) into 7-hydroxy-trifluoromethyl coumarin. The CYP2D6 protein converts 3-[2-(N,N-diethyl-N-methylamino)ethyl]-7-methoxy-4-methylcoumarin (AMMC) into 3-[2-(N,N-diethylamino)ethyl]-7-hydroxy-4-methylcoumarin hydrochloride and the CYP2C9 protein converts 7-Methoxy-4-trifluoromethyl coumarin (MFC) into 7-hydroxy-trifluoromethyl coumarin. Compounds inhibiting the enzymatic reaction will result in a decrease of fluoresent signal (see example C.5).

### Example C.1: In Vitro Assay for Inhibition of histone deaceylase:

The HDAC Fluorescent Activity Assay/Drug Discovery Kit of Biomol (cat.No: AK-500-0001) was used. The HDAC Fluorescent Activity Assay is based on the Fluor de Lys Fluorogenic Histone deAcetylase Lysyl) substrate and developer combination. The Fluor de Lys substrate, comprises an acetylated lysine side chain. Deacetylation of the substrate sensitizes the substrate so that, in the second step, treatment with the Fluor de Lys developer produces a fluorophore.

HeLa nuclear extracts (supplier: Biomol) were incubated at 60 µg/ml with 75 µM of substrate. The Fluor de Lys substrate was added in a buffer containing 25 mM Tris, 137 mM NaCl, 2.7 mM KCl and 1 mM MgCl₂.6H₂O at pH 7.4. After 30 min, 1 volume of the developer was added. The fluorophore was excited with 355 nm light and the emitted light (450 nm) was be detected on a fluorometric plate reader.

For each experiment, controls (containing HeLa nuclear extract and buffer), a blank incubation (containing buffer but no HeLa nuclear extract) and samples (containing compound dissolved in DMSO and further diluted in buffer and HeLa nuclear extract) were run in parallel. In first instance, compounds were tested at a concentration of 10⁻⁵M. When the compounds showed activity at 10⁻⁵M, a concentration-response curve was made wherein the compounds were tested at concentrations between 10-⁵M and 10⁻⁹M. All sample were tested 4 times. In each test the blank value was substracted from both the control and the sample values. The control sample represented 100% of substrate deactylation. For each sample the fluorescence was expressed as a percentage of the mean value of the controls. When appropriate IC₅₀-values (concentration of the drug, needed to reduce the amount of metabolites to 50% of the control) were computed using probit analysis for graded data. Herein the effects of test compounds are expressed as pIC₅₀ (the negative log value of the IC₅₀-value) (see Table F-3).

### Example C.2: Determination of antiproliferative activity on A2780 cells

All compounds tested were dissolved in DMSO and further dilutions were made in culture medium. Final DMSO concentrations never exceeded 0.1 % (v/v) in cell proliferation assays. Controls contained A2780 cells and DMSO without compound and blanks contained DMSO but no cells. MTT was dissolved at 5 mg/ml in PBS. A glycine buffer comprised of 0.1 M glycine and 0.1 M NaCl buffered to pH 10.5 with NaOH.(1 N) was prepared (all reagents were from Merck).

The human A2780 ovarian carcinoma cells (a kind gift from Dr. T.C. Hamilton [Fox Chase Cancer Centre, Pennsylvania, USA]) were cultured in RPMI 1640 medium supplemented with 2 mM L-glutamine, 50 µg/ml gentamicin and 10 % fetal calf serum. Cells were routinely kept as monolayer cultures at 37°C in a humidified 5 % CO₂ atmosphere. Cells were passaged once a week using a trypsin/EDTA solution at a split ratio of 1:40. All media and supplements were obtained from Life Technologies. Cells were free of mycoplasma contamination as determined using the Gen-Probe Mycoplasma Tissue Culture kit (supplier: BioMérieux).

Cells were seeded in NUNC^{™} 96-well culture plates (Supplier: Life Technologies) and allowed to adhere to the plastic overnight. Densities used for plating were 1500 cells per well in a total volume of 200 µl medium. After cell adhesion to the plates, medium was changed and drugs and/or solvents were added to a final volume of 200 µl. Following four days of incubation, medium was replaced by 200 µl fresh medium and cell density and viability was assessed using an MTT-based assay. To each well, 25 µl MTT solution was added and the cells were further incubated for 2 hours at 37°C. The medium was then carefully aspirated and the blue MTT-formazan product was solubilized by addition of 25 µl glycine buffer followed by 100 µl of DMSO. The microtest plates were shaken for 10 min on a microplate shaker and the absorbance at 540 nm was measured using an Emax 96-well spectrophotometer (Supplier: Sopachem). Within an experiment, the results for each experimental condition are the mean of 3 replicate wells. For initial screening purposes, compounds were tested at a single fixed concentration of 10⁻⁶ M. For active compounds, the experiments were repeated to establish full concentration-response curves. For each experiment, controls (containing no drug) and a blank incubation (containing no cells or drugs) were run in parallel. The blank value was subtracted from all control and sample values. For each sample, the mean value for cell growth (in absorbance units) was expressed as a percentage of the mean value for cell growth of the control. When appropriate, IC₅₀-values (concentration of the drug, needed to reduce cell growth to 50% of the control) were computed using probit analysis for graded data (Finney, D.J., Probit Analyses, 2nd Ed. Chapter 10, Graded Responses, Cambridge University Press, Cambridge 1962). Herein the effects of test compounds are expressed as pIC₅₀ (the negative log value of the IC₅₀-value)(see Table F-3).

### Example C.3: Solubility/Stability

The solubility of a compound, at different pH's, can be measured with the use of a chemiluminescent nitrogen detector

### Example C.4: p21 induction capacity

### Example C:4: a: p21 enzyme linked immunosorbent assay

The following protocol has been applied to determine the p21 protein expression level in human A2780 ovarian carcinoma cells. The A2780 cells (20000 cells /180 µl) were seeded in 96 microwell plates in RPMI 1640 medium supplemented with 2 mM L-glutamine, 50 µg/ml gentamicin and 10 % fetal calf serum. 24 hours before the lysis of the cells, compounds were added at final concentrations of 10⁻⁵, 10⁻⁶, 10⁻⁷ and 10⁻⁸ M. All compounds tested were dissolved in DMSO and further dilutions were made in culture medium. 24 hours after the addition of the compound, the supernatants were removed from the cells. Cells were washed with 200 µl ice-cold PBS. The wells were aspirated and 30 µl of lysisbuffer (50 mM Tris.HCl (pH 7.6), 150 mM NaCl, 1 % Nonidet p40 and 10 % glycerol) was added. The plates were incubated overnight at -70 °C.

The appropriate number of microtiter wells were removed from the foil pouch and placed into an empty well holder. A working solution (1x) of the Wash Buffer (20x plate wash concentrate: 100 ml 20-fold concentrated solution of PBS and surfactant. Contains 2 % chloroacetamide) was prepared. The lyophilised p21 WAF standard was reconstituted with distilled H₂O and further diluted with sample diluent (provided in the kit)

The samples were prepared by diluting them 1:4 in sample diluent. The samples (100 µl) and the p21 WAF1 standards (100 µl) were pipetted into the appropriate wells and incubated at room temperature for 2 hours. The wells were washed 3 times with 1x wash buffer and then 100 µl of detector antibody reagent (a solution of biotinylated monoclonal p21 WAF1 antibody) was pipetted into each well. The wells were incubated at room temperature for 1 hour and then washed three times with 1x wash buffer. The 400x conjugate (peroxidase streptavidine conjugate: 400-fold concentrated solution) was diluted and 100 µl of the 1x solution was added to the wells. The wells were incubated at room temperature for 30 min and then washed 3 times with 1x wash buffer and 1 time with distilled H₂O. Substrate solution (chromogenic substrate)(100 µl) was added to the wells and the wells were incubated for 30 minutes in the dark at room temperature. Stop solution was added to each well in the same order as the previously added substrate solution. The absorbance in each well was measured using a spectrophotometric plate reader at dual wavelengths of 450/595 nm.

For each experiment, controls (containing no drug) and a blank incubation (containing no cells or drugs) were run in parallel. The blank value was substracted from all control and sample values. For each sample, the value for p21WAF1 induction (in absorbance units) was expressed as the percentage of the value for p21WAF1 present in the control. Percentage induction higher than 130 % was defined as significant induction. Eleven compounds were tested and showed significant induction.

### Example C.4.b.: cellular method

A2780 cells (ATCC) were cultivated in RPMI 1640 medium supplemented with 10% FCS, 2 mM L-glutamine and gentamycine at 37°C in a humidified incubator with 5%CO₂. All cell culture solutions are provided by Gibco-BRL (Gaithersburg, MD). Other materials are provided by Nunc.

Genomic DNA was extracted from proliferating A2780 cells and used as template for nested PCR isolation of the p21 promoter. The first amplification was performed for 20 cycles at an annealing temperature of 55°C using the oligonucleotide pair GAGGGCGCGGTGCTTGG and TGCCGCCGCTCTCTCACC with the genomic DNA as template. The resulting 4.5 kb fragment containing the -4551 to +88 fragment relative to the TATA box was re-amplified with the oligonucleotides TCGGGTACCGAGGGCGCGGTGCTTGG and ATACTCGAGTGCCGCCGCTCTCTCACC for 20 cycles with annealing at 88°C resulting in a 4.5 kb fragment and subsequently with the oligonucleotide pair TCGGGTACCGGTAGATGGGAGCGGATAGACACATC and ATACTCGAGTGCCGCCGCTCTCTCACC for 20 cycles with annealing at 88°C resulting in a 1.3 kb fragment containing the -1300 to +88 fragment relative to the TATA box. The restriction sites XhoI and KpnI present in the oligonucleotides (underlined sequence) were used for subcloning.

The luciferase reporter was removed from the pGL3-basic and replaced by the ZsGreen reporter (from the pZsGreenl-N1 plasmid) at KpnI and XbaI restriction sites. pGL3-basic-ZsGreen-1300 was constructed via insertion of the above mentioned 1.3 kb fragment of the human p21 promoter region into pGL3-basic-ZsGreen at the XhoI and KpnI sites. All restriction enzymes are provided by Boehringer Manheim (Germany). A2780 cells were plated into a 6-well plate at a density of 2x10⁵ cells, incubated for 24 hours, and transfected with 2 ug of pGL3-basic-ZsGreen-1300 and 0.2 ug of pSV2neo vector by using Lipofectamine 2000 (Invitrogen, Brussels, Belgium) as described by manufacturer. The transfected cells were selected for 10 days with G418 (Gibco-BRL, Gaithersburg, MD) and single cell suspensions were grown. After three weeks, single clones were obtained.

The A2780 selected clones were expanded and seeded at 10000 cells per well into 96-well plates. 24 hours after seeding, the cells were treated for an additional 24 hours with compounds (affecting sp1 sites in the proximal p21 promoter region). Subsequently, cells were fixed with 4% PFA for 30' and counterstained with Hoechst dye. The p21 promoter activation leading to ZsGreen production and thus fluorescence, was monitored by the Ascent Fluoroskan (Thermo Labsystems, Brussels, Belgium).

For each experiment, controls (containing no drug) and a blank incubation (containing no cells or drugs) were run in parallel. The blank value was substracted from all control and sample values. For each sample, the value for p21 induction was expressed as the percentage of the value for p21 present in the control. Percentage induction higher than 130 % was defined as significant induction.

Eleven compounds were tested and showed significant induction.

### Example C.5: P450 inhibiting capacity

All compounds tested were dissolved in DMSO (5 mM) and a further dilution to 5 10⁻⁴ M was made in acetonitrile. Further dilutions were made in assay buffer (0.1 M NaK phosphate buffer pH 7.4) and the final solvent concentration was never higher than 2 %.

The assay for the CYP3A4 protein comprises per well 15 pmol P450/mg protein (in 0.01M NaKphosphate buffer +1.15% KCl), an NADPH generating system (3.3 mM Glucose-6-phosphate, 0.4 U/ml Glucose-6-phosphate dehydrogenase, 1.3 mM NADP and 3.3 mM MgCl₂.6H₂O in assay buffer) and compound in a total assay volume of 100 µl. After a 5 min pre-incubation at 37 °C the enzymatic reaction was started with the addition of 150 µM of the fluoresent probe substrate BFC in assay buffer. After an incubation of 30 minutes at room temperature the reaction was terminated after addition of 2 volumes of acetonitrile. Fluorescent determinations were carried out at an excitation wavelength of 405 nm and an emission wavelength of 535 nm. Ketoconazole (IC₅₀-value =3X10⁻⁸M) was included as reference compound in this experiment.

The assay for the CYP2D6 protein comprises per well 6 pmol P450/mg protein (in 0.0 1 M NaKphosphate buffer +1.15% KCl), an NADPH generating system (0.41 mM Glucose-6-phosphate, 0.4 U/ml Glucose-6-phosphate dehydrogenase, 0.0082 mM NADP and 0.41 mM MgCl₂.6H₂O in assay buffer) and compound in a total assay volume of 100 µl After a 5 min pre-incubation at 37 °C the enzymatic reaction was started with the addition of 3 µM of the fluoresent probe substrate AMMC in assay buffer. After an incubation of 45 minutes at room temperature the reaction was terminated after addition of 2 volumes of acetonitrile. Fluorescent determinations were carried out at an excitation wavelength of 405 nm and an emission wavelength of 460 nm. Quinidine (IC₅₀-value < 5 X 10⁻⁸ M) was included as reference compound in this experiment.

The assay for the CYP2C9 protein comprises per well 15 pmol P450/mg protein (in 0.01 M NaKphosphate buffer + 1.15% KCl), an NADPH generating system (3.3 mM Glucose-6-phosphate, 0.4 U/ml Glucose-6-phosphate dehydrogenase, 1.3 mM NADP and 3.3 mM MgCl₂.6H₂O in assay buffer) and compound in a total assay volume of 100 µl. After a 5 min pre-incubation at 37 °C the enzymatic reaction was started with the addition of 200 µM of the fluoresent probe substrate MFC in assay buffer. After an incubation of 30 minutes at room temperature the reaction was terminated after addition of 2 volumes of acetonitrile. Fluorescent determinations were carried out at an excitation wavelength of 405 nm and an emission wavelength of 535 nm. Sulfaphenazole (IC₅₀-value = 6.8 X 10⁻⁷ M) was included as reference compound in this experiment.

For initial screening purposes, compounds were tested at a single fixed concentration of 1 X 10⁻⁵ M. For active compounds, the experiments were repeated to establish full concentration-response curves. For each experiment, controls (containing no drug) and a blank incubation (containing no enzyme or drugs) were run in parallel. All compounds were assayed in quadruplicate. The blank value was subtracted from all control and sample values. For each sample, the mean value of P450 activity of the sample (in relative fluorescence units) was expressed as a percentage of the mean value of P450 activity of the control. Percentage inhibition was expressed as 100% minus the mean value of P450 activity of the sample. When appropriate, IC₅₀-values (concentration of the drug, needed to reduce P450 activity to 50% of the control) were calculated.

**Table F-3: lists the results of the compounds that were tested according to example C.1 and C.2,**

| Co. No. | Enzyme activity pIC50 | Cellular activity pIC50 |
|---|---|---|
| 1 | 7.1 | 6.0 |
| 2 | 7.5 | 6.9 |
| 3 | 7.8 | 7.1 |
| 4 | 7.6 | 6.2 |
| 5 | 7.1 | 6.2 |
| 6 | 7.2 | 6.3 |
| 7 | 6.9 | 6.5 |
| 8 | 6.4 | 6.7 |
| 9 | 7.3 | 6.5 |
| 10 | 7.2 | 5.6 |
| 11 | 7.3 | 6.2 |
| 12 | 7.5 | 6.2 |
| 13 | 7.3 | 6.1 |
| 14 | 6.8 | 5.2 |
| 15 | 7.3 | 5.9 |
| 16 | 7.2 | 5.9 |
| 17 | 7.4 | 6.1 |
| 18 | 7.2 | 5.9 |
| 19 | 7.4 | 6.3 |
| 20 | 7.3 | 6.3 |
| 21 | 7.4 | 6.3 |
| 22 | 7.2 | 5.3 |
| 23 | 7.2 | 5.9 |
| 24 | 7.4 | 6.2 |
| 25 | 7.4 | 6.2 |
| 26 | 7.4 | 6.3 |
| 27 | 7.5 | 6.2 |
| 28 | 7.6 | 6.2 |
| 29 | 7.7 | 6.2 |
| 30 | 7.5 | 6.2 |
| 31 | 7.7 | 6.2 |
| 32 | 6.6 | 6.0 |
| 33 | 7.0 | 6.0 |
| 34 | 7.2 | 6.2 |
| 35 | 7.9 | 6.3 |
| 36 | 7.0 | 5.8 |

### D. Composition example: Film-coated tablets

### Preparation of tablet core

A mixture of 100 g of a compound of formula (I), 570 g lactose and 200 g starch is mixed well and thereafter humidified with a solution of 5 g sodium dodecyl sulphate and 10 g polyvinyl-pyrrolidone in about 200 ml of water. The wet powder mixture is sieved, dried and sieved again. Then there is added 100 g microcrystalline cellulose and 15 g hydrogenated vegetable oil. The whole is mixed well and compressed into tablets, giving 10.000 tablets, each comprising 10 mg of a compound of formula (I).

### Coating

To a solution of 10 g methyl cellulose in 75 ml of denaturated ethanol there is added a solution of 5 g of ethyl cellulose in 150 ml of dichloromethane. Then there are added 75 ml of dichloromethane and 2.5 ml 1,2,3-propanetriol 10 g of polyethylene glycol is molten and dissolved in 75 ml of dichloromethane. The latter solution is added to the former and then there are added 2.5 g of magnesium octadecanoate, 5 g of polyvinyl-pyrrolidone and 30 ml of concentrated colour suspension and the whole is homogenated. The tablet cores are coated with the thus obtained mixture in a coating apparatus.

## Claims

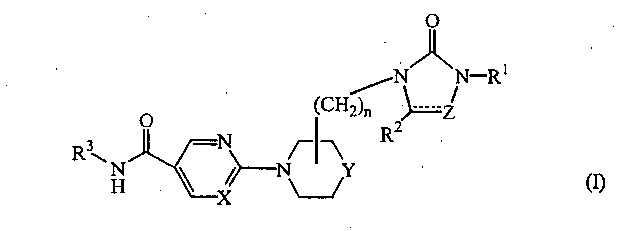

1. the N-oxide forms, the pharmaceutically acceptable addition salts and the stereochemically isomeric forms thereof, wherein
each X is independently N or CH;
each Y is independently O, CH or CH₂ and when Y is CH then the substituent is attached to the Y atom of the ring structure;
each Z is independently C=O, CH₂ or CH and when Z is CH then the dotted line is a bond;
n is 0 or 1 and when n is 0 then a direct bond is intended;
R¹ is phenyl, naphtalenyl, heterocyclyl, phenylC₁₋₆alkyl, naphtalenylC₁₋₆alkyl, heterocyclylC₁₋₆alkyl; wherein each of said phenyl, naphtalenyl or heterocyclyl is optionally substituted with one, two or three substituents each independently selected from hydrogen, halo, C₁₋₆alkyl, C₁₋₆alkyloxy, polyhaloC₁₋₆alkyl, phenyl, phenyloxy, cyano, C₁₋₆alkylcarbonylamino or two substituents taken together can form the bivalent radical -O-(CH₂)₂-O-(CH₂)₂- O-(CH₂)₂- O-(CH2)₂- O-;
R² is hydrogen, C₁₋₆alkyl or phenyl wherein each phenyl is optionally substituted with one or two substituents each independently selected from hydrogen, halo, C₁₋₆alkyl, C₁₋₆alkyloxy, phenyloxy or cyano;
R³ is hydroxy or a radical of formula (a-1) 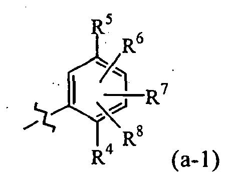 wherein
R⁴ is hydroxy or -NH₂;
R⁵ is hydrogen, thienyl, furanyl or phenyl and each thienyl, furanyl or phenyl can optionally be substituted with halo, amino, nitro, cyano, hydroxy, phenyl, C₁₋₆alkyl, (diC₁₋₆alkyl)amino, C₁₋₆alkyloxy, phenylC₁₋₆alkyloxy, hydroxyC₁₋₆alkyl, C₁₋₆alkyloxycarbonyl, hydroxycarbonyl ,C₁₋₆alkylcarbonyl, polyhaloC₁₋₆alkyloxy, polyhaloC₁₋₆alkyl, C₁₋₆alkylsulfonyl, hydroxycarbonylC₁₋₆alkyl, C₁₋₆alkylcarbonylamino, aminosulfonyl, aminosulfonylC₁₋₆alkyl, isoxazolyl, aminocarbonyl, phenylC₂₋₆alkenyl, phenylC₃₋₆alkynyl or pyridinylC₃₋₆alkynyl; R⁶, R⁷ and R⁸ are each independently hydrogen, -NH₂, nitro, furanyl, halo, C₁₋₆alkyl, C₁₋₆alkyloxy, trifluoromethyl, thienyl, phenyl, C₁₋₆alkylcarbonylamino, aminocarbonylC₁₋₆alkyl or -C=C-CH₂-R⁹;
wherein R⁹ is hydrogen, C₁₋₆alkyl, hydroxy, amino or C₁₋₆alkyloxy; and
heterocyclyl in the above is furanyl, thienyl, pyrrolyl, pyrrolinyl, pyrolidinyl, dioxolyl, oxazolyl, thiazolyl, imidazolyl, imidazolinyl, imidazolidinyl, pyrazolyl, pyrazolinyl, pyrazolidinyl, isoxazolyl, isothiazolyl, oxadiazolyl, triazolyl, thiadiazolyl, pyranyl, pyridinyl, piperidinyl, dioxanyl, morpholinyl, dithianyl, thiomorpholinyl, pyridazinyl, pyrimidinyl, pyrazinyl, piperazinyl, triazinyl, trithianyl, indolizinyl, indolyl, indolinyl, benzofuranyl, benzothiophenyl, indazolyl, benzimidazolyl, benzthiazolyl, purinyl, quinolizinyl, quinolinyl, cinnolinyl, phthlazinyl, quinazolinyl, quinoxalinyl or naphthyridinyl.

2. A compound according to claim 1 wherein
each X is N; each Y is independently O or CH;n is 1; R¹ is phenyl, naphtalenyl or heterocyclylC₁₋₆alkyl wherein each of said phenyl, naphtalenyl or heterocyclylC₁₋₆alkyl is optionally substituted with one, two or three substituents each independently selected from hydrogen, halo,
C₁₋₆alkyl, C₁₋₆alkyloxy, polyhaloC₁₋₆alkyl, phenyl, phenyloxy, or cyano, or two substituents taken together can form the bivalent radical -O-(CH₂)₂-O-(CH₂)₂- O-(CH₂)₂- O-(CH₂)₂- O-; R² is hydrogen, C₁₋₆alkyl or phenyl and R³ is hydroxyl.

3. A compound according to claim 1 and 2 wherein
each X is N; each Y is CH; each Z is CH; R¹ is phenyl or heterocyclylC₁₋₆alkyl wherein each of said phenyl or heterocyclylC₁₋₆alkyl is optionally substituted with one, two or three substituents each independently selected from hydrogen, C₁₋₆alkyl, phenyl or phenyloxy;R² is hydrogen and R³ is hydroxy.

4. A compound according to claim 1, 2 and 3 wherein said compound is compound No. 3, compound No.2 and compound No 35.
| | |
|---|---|
| 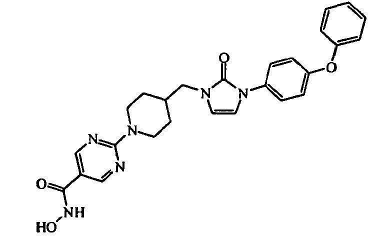 | 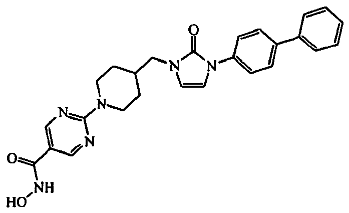 |
| Co. No. 2 | Co. No. 3 |
| 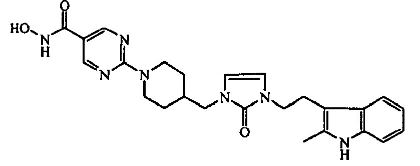 | |
| Co. No. 35 | |

5. A pharmaceutical composition comprising pharmaceutically acceptable carriers and as an active ingredient a therapeutically effective amount of a compound as claimed in claim 1 to 4.

6. A process of preparing a pharmaceutical composition as claimed in claim 5 wherein the pharmaceutically acceptable carriers and a compound as claimed in claim 1 to 4 are intimately mixed.

7. A compound as claimed in any of claims 1 to 4 for use as a medicine.

8. Use of a compound as claimed in any of claims 1 to 4 for the manufacture of a medicament for the treatment of proliferative diseases.

9. A combination of an anti-cancer agent and a HDAC inhibitor as claimed in any of claims 1 to 4.

10. A process for preparing a compound as claimed in claim 1, **characterized by**
a) reacting an intermediate of formula (II) with an appropriate acid in a suitable solvent, yielding a compound of formula (I), wherein R³ is hydroxy, herein referred to as compounds of formula (I-a), 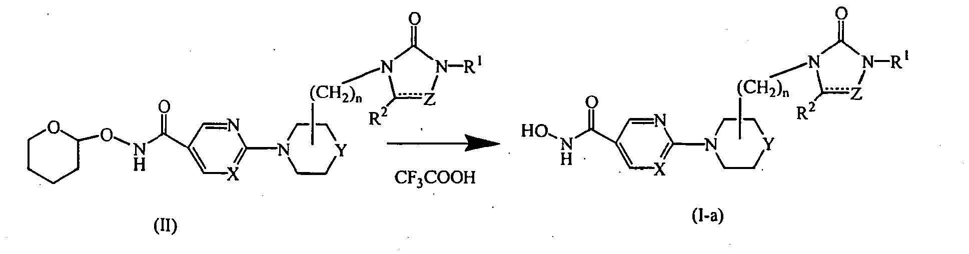
b) reacting an intermediate of formula (XV) with tin(II) chloride hydrate or with hydrogen in the presence of 10 % palladium on charcoal, in a suitable solvent with the formation of a compound of formula (I), wherein R³ is a radical of formula (a-1) and R⁴ is -NH₂, herein referred to as compounds of formula (I-b), 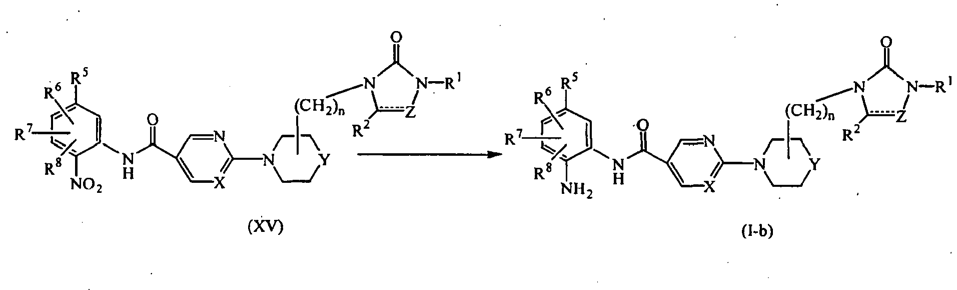
c) reacting an intermediate of formula (XVI) with an appropriate acid in a suitable solvent, with the formation of a compound of formula (I-b), 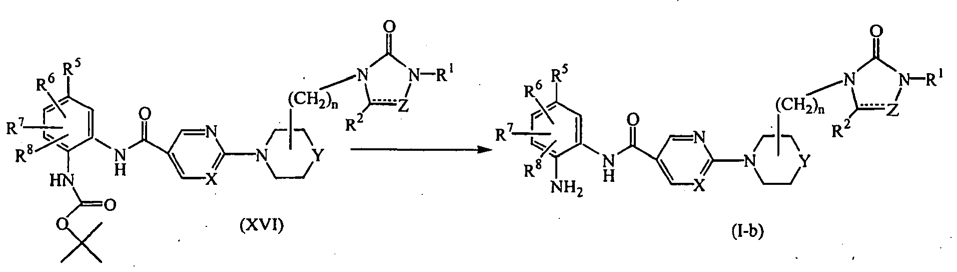
d) reacting an intermediate of formula (XVII), wherein TBDMS in the means *tert*-butyl(dimethyl)silanyl, with tetrabutylammonium fluoride in a suitable solvent, with the formation of a compound of formula (I), wherein R³ is a radical of formula (a-1) and R⁴ is hydroxy, herein referred to as compounds of formula (I-c).

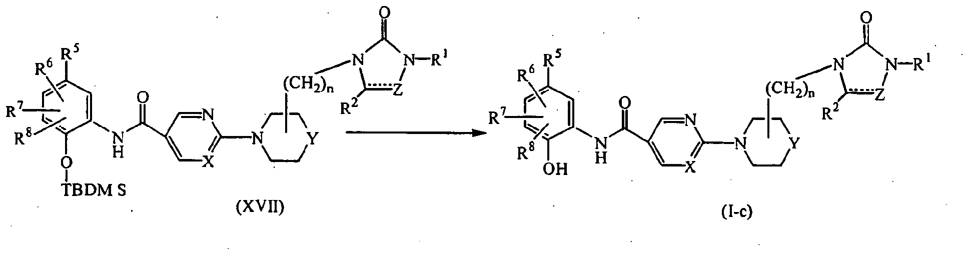

## Patentansprüche

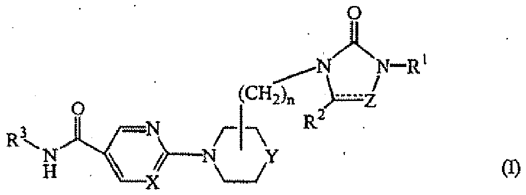

1. die N-Oxid-Formen, die pharmazeutisch unbedenklichen Additionssalze und die stereochemisch isomeren Formen davon, wobei
jedes X unabhängig für N oder CH steht;
jedes Y unabhängig für O, CH oder CH₂ steht und dann, wenn Y für CH steht, der Substituent an das Y-Atom der Ringstruktur gebunden ist;
jedes Z unabhängig für C=O, CH₂ oder CH steht und dann, wenn Z für CH steht, die gestrichelte Linie eine Bindung ist;
n für 0 oder 1 steht und dann, wenn n für 0 steht, eine direkte Bindung gemeint ist;
R¹ für Phenyl, Naphthalinyl, Heterocyclyl, Phenyl-C₁₋₆-alkyl, Naphthalinyl-C₁₋₆-alkyl oder Heterocyclyl-C₁₋₆-alkyl steht; wobei jedes Phenyl, Naphthalinyl oder Heterocyclyl gegebenenfalls durch einen, zwei oder drei jeweils unabhängig unter Wasserstoff, Halogen, C₁₋₆-Alkyl, C₁₋₆-Alkyloxy, Polyhalogen-C₁₋₆-alkyl, Phenyl, Phenyloxy, Cyano, C₁₋₆-Alkylcarbonylamino ausgewählte Substituenten substituiert ist oder zwei Substituenten zusammengenommen den zweiwertige Rest -O-(CH₂)₂-O-(CH₂)₂-O-(CH₂)₂-O-(CH₂)₂-O- bilden können;
R² für Wasserstoff, C₁₋₆-Alkyl oder Phenyl steht, wobei jedes Phenyl gegebenenfalls durch einen oder zwei jeweils unabhängig unter Wasserstoff, Halogen, C₁₋₆-Alkyl, C₁₋₆-Alkyloxy, Phenyloxy oder Cyano ausgewählte Substituenten substituiert ist; R³ für Hydroxy oder einen Rest der Formel (a-1) 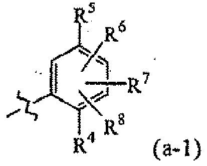 steht, wobei
R⁴ für Hydroxy oder -NH₂ steht;
R⁵ für Wasserstoff, Thienyl, Furanyl oder Phenyl steht und jedes Thienyl, Furanyl oder Phenyl gegebenenfalls durch Halogen, Amino, Nitro, Cyano, Hydroxy, Phenyl, C₁₋₆-Alkyl, (Di-C₁₋₆-alkyl)amino, C₁₋₆-Alkyloxy, Phenyl-C₁₋₆-alkyloxy, Hydroxy-C₁₋₆-alkyl, C₁₋₆-Alkyloxycarbonyl, Hydroxycarbonyl, C₁₋₆-Alkylcarbonyl, Polyhalogen-C₁₋₆-alkyloxy, Polyhalogen-C₁₋₆-alkyl, C₁₋₆-Alkylsulfonyl, Hydroxycarbonyl-C₁₋₆-alkyl, C₁₋₆-Alkylcarbonylamino, Aminosulfonyl, Aminosulfonyl-C₁₋₆-alkyl, Isoxazolyl, Aminocarbonyl, Phenyl-C₂₋₆-alkenyl, Phenyl-C₃₋₆-alkinyl oder Pyridinyl-C₃₋₆-alkinyl substituiert sein kann;
R⁶, R⁷ und R⁸ jeweils unabhängig für Wasserstoff, -NH₂, Nitro, Furanyl, Halogen, C₁₋₆-Alkyl, C₁₋₆-Alkyloxy, Trifluormethyl, Thienyl, Phenyl, C₁₋₆-Alkylcarbonylamino, Aminocarbonyl-C₁₋₆-alkyl oder -C=C-CH₂-R⁹ stehen;
wobei R⁹ für Wasserstoff, C₁₋₆-Alkyl, Hydroxy, Amino oder C₁₋₆-Alkoxy steht; und
Heterocyclyl im obigen Text für Furanyl, Thienyl, Pyrrolyl, Pyrrolinyl, Pyrrolidinyl, Dioxolyl, Oxazolyl, Thiazolyl, Imidazolyl, Imidazolinyl, Imidazolidinyl, Pyrazolyl, Pyrazolinyl, Pyrazolidinyl, Isoxazolyl, Isothiazolyl, Oxadiazolyl, Triazolyl, Thiadiazolyl, Pyranyl, Pyridinyl, Piperidinyl, Dioxanyl, Morpholinyl, Dithianyl, Thiomorpholinyl, Pyridazinyl, Pyrimidinyl, Pyrazinyl, Piperazinyl, Triazinyl, Trithianyl, Indolizinyl, Indolyl, Indolinyl, Benzofuranyl, Benzothiophenyl, Indazolyl, Benzimidazolyl, Benzthiazolyl, Purinyl, Chinolizinyl, Chinolinyl, Cinnolinyl, Phthalazinyl, Chinazolinyl, Chinoxalinyl oder Naphthyridinyl steht.

2. Verbindung nach Anspruch 1, wobei jedes X für N steht; jedes Y unabhängig für 0 oder CH steht; n für 1 steht; R¹ für Phenyl, Naphthalinyl oder Heterocyclyl-C₁₋₆-alkyl steht, wobei jedes Phenyl, Naphthalinyl oder Heterocyclyl-C₁₋₆-alkyl gegebenenfalls durch einen, zwei oder drei jeweils unabhängig unter Wasserstoff, Halogen, C₁₋₆-Alkyl, C₁₋₆-Alkyloxy, Polyhalogen-C₁₋₆-alkyl, Phenyl, Phenyloxy oder Cyano ausgewählte Substituenten substituiert ist oder zwei Substituenten zusammengenommen den zweiwertigen Rest -O-(CH₂)₂-O-(CH₂)₂-O-(CH₂)₂-O-(CH₂)₂-O- bilden können; R² für Wasserstoff, C₁₋₆-Alkyl oder Phenyl steht und R³ für Hxdroxyl steht.

3. Verbindung nach Anspruch 1 und 2, wobei jedes X für N steht; jedes Y für CH steht; jedes Z für CH steht; R¹ für Phenyl oder Heterocyclyl-C₁₋₆-alkyl steht, wobei jedes Phenyl oder Heterocyclyl-C₁₋₆-alkyl gegebenenfalls durch einen, zwei oder drei jeweils unabhängig unter Wasserstoff, C₁₋₆-Alkyl, Phenyl oder Phenyloxy ausgewählte Substituenten substituiert ist; R² für Wasserstoff steht und R³ für Hxdroxy steht.

4. Verbindung nach Anspruch 1, 2 oder 3, wobei es sich bei der Verbindung um Verbindung Nr. 3, Verbindung Nr. 2 und Verbindung Nr. 35 handelt:
| | |
|---|---|
| 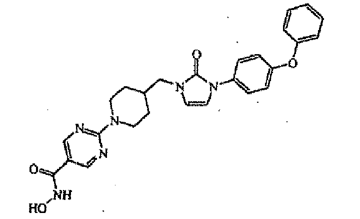 | 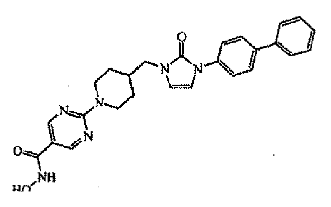 |
| Verb. Nr. 2 | Verb. Nr. 3 |
| 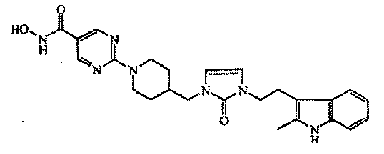 | |
| Verb. Nr. 35 | |

5. Pharmazeutische Zusammensetzung, enthaltend pharmazeutisch unbedenkliche Träger und als Wirkstoff eine therapeutisch wirksame Menge einer Verbindung nach einem der Ansprüche 1 bis 4.

6. Verfahren zur Herstellung einer pharmazeutischen Zusammensetzung nach Anspruch 5, bei dem man die pharmazeutisch unbedenklichen Träger und eine Verbindung nach einem der Ansprüche 1 bis 4 innig vermischt.

7. Verbindung nach einem der Ansprüche 1 bis 4 zur Verwendung als Medizin.

8. Verwendung einer Verbindung nach einem der Ansprüche 1 bis 4 zur Herstellung eines Arzneimittels zur Behandlung von proliferativen Erkrankungen.

9. Kombination aus einem Antikrebsmittel und einem HDAC-Inhibitor nach einem der Ansprüche 1 bis 4.

10. Verfahren zur Herstellung einer Verbindung nach Anspruch 1, **dadurch gekennzeichnet, dass** man
a) ein Zwischenprodukt der Formel (II) in einem geeigneten Lösungsmittel mit einer entsprechenden Säure zu einer Verbindung der Formel (I), worin R³ für Hydroxy steht, hier als Verbindungen der Formel (I-a) bezeichnet, umsetzt, 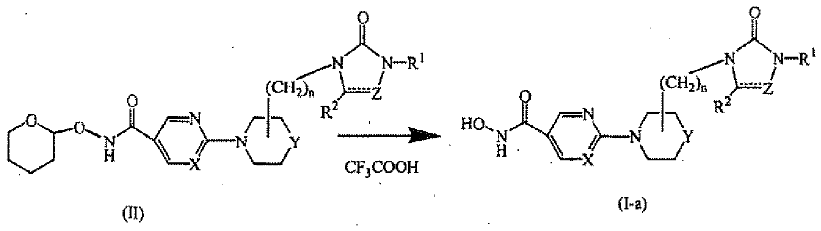
b) ein Zwischenprodukt der Formel (XV) in einem geeigneten Lösungsmittel mit Zinn(II)-chlorid-hydrat oder mit Wasserstoff in Gegenwart von 10% Palladium auf Kohle zu einer Verbindung der Formel (I), worin R³ für einen Rest der Formel (a-1) steht und R⁴ für -NH₂ steht, hier als Verbindungen der Formel (I-b) bezeichnet, umsetzt, 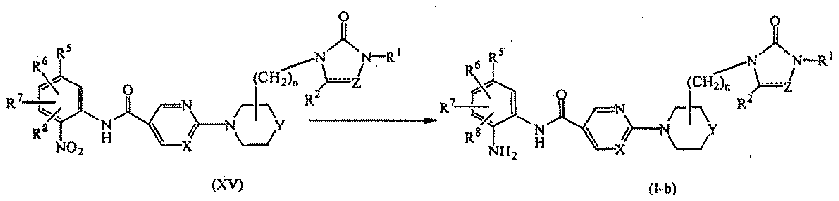
c) ein Zwischenprodukt der Formel (XVI) in einem geeigneten Lösungsmittel mit einer entsprechenden Säure zu einer Verbindung der Formel (I-b) umsetzt, 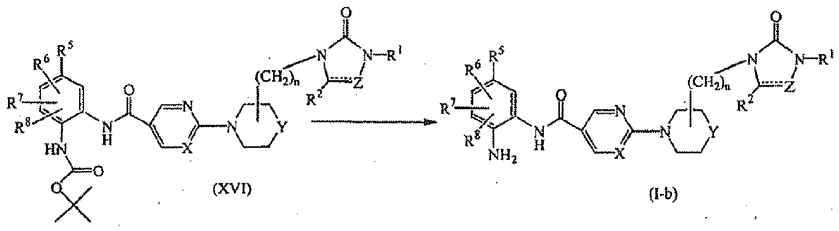
d) ein Zwischenprodukt der Formel (XVII), worin TBDMS für tert.-Butyl(dimethyl)silanyl steht, in einem geeigneten Lösungsmittel mit Tetrabutylammoniumfluorid zu einer Verbindung der Formel (I), worin R³ für einen Rest der Formel (a-1) steht und R⁴ für Hydroxy steht, hier als Verbindungen der Formel (I-c) bezeichnet, umsetzt.

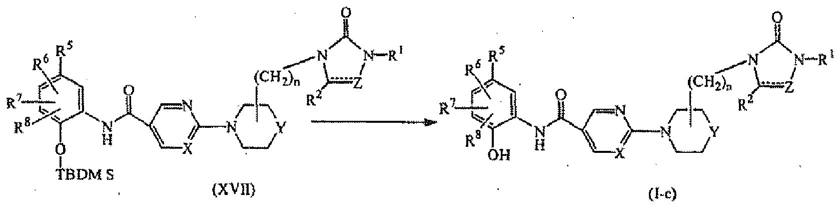

## Revendications

1. Composé de formule 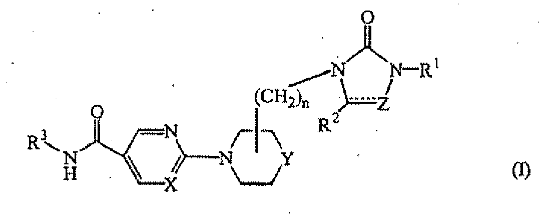 les formes N-oxydes, les sels d'addition pharmaceutiquement acceptables et les formes stéréochimiquement isomères de celui-ci, dans laquelle
chaque X est indépendamment N ou CH ;
chaque Y est indépendamment, O, CH ou CH₂ et lorsque Y est CH, alors le substituant est lié à l'atome Y de la structure cyclique ;
chaque Z est indépendamment C=O, CH₂ ou CH et lorsque Z est CH, alors la ligne en pointillés est une liaison ;
n est 0 ou 1 et lorsque n est 0, alors une liaison directe est visée ;
R¹ est phényle, naphtalinyle, hétérocyclyle, phényl-C₁₋₆-alkyle, naphthalinyl-C₁₋₆-alkyl ou hétérocyclyl-C₁₋₆-alkyle ; où chacun desdits phényle, naphtalinyle ou hétérocyclyle est éventuellement substitué par un, deux ou trois substituants choisis chacun indépendamment parmi hydrogène, halogéno, C₁₋₆-alkyle, C₁₋₆-alkyloxy, polyhalogéno-C₁₋₆-alkyle, phényle, phényloxy, cyano, C₁₋₆-alkylcarbonylamino ou deux substituants pris ensemble peuvent former un radical bivalent -O-(CH₂)₂-O-(CH₂)₂-O-(CH₂)₂-O-(CH₂)₂-O- ;
R² est hydrogène, C₁₋₆-alkyle ou phényle, chaque phényle étant éventuellement substitué par un ou deux substituants choisis chacun indépendamment parmi hydrogène, halogéno, C₁₋₆-alkyle, C₁₋₆-alkyloxy, phényloxy ou cyano ;
R³ est hydroxy ou un radical de formule (a-1) 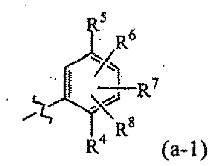 dans laquelle
R⁴ est hydroxy ou -NH₂ ;
R⁵ est hydrogène, thiényle, furanyle ou phényle et chaque thiényle, furanyle ou phényle peut éventuellement être substitué par halogéno, amino, nitro, cyano, hydroxy, phényle, C₁₋₆-alkyle, (di-C₁₋₆-alkyl) amino, C₁₋₆-alkyloxy, phényl-C₁₋₆-alkyloxy, hydroxy-C₁₋₆-alkyle, C₁₋₆-alkyloxycarbonyle, hydroxycarbonyle, C₁₋₆-alkylcarbonyle, polyhalogéno-C₁₋₆-alkyloxy, polyhalogéno-C₁₋₆-alkyle, C₁₋₆-alkylsulfonyle, hydroxycarbonyl-C₁₋₆-alkyle, C₁₋₆-alkylcarbonylamino, aminosulfonyle, aminosulfonyl-C₁₋₆-alkyle, isoxazolyle, aminocarbonyle, phényl-C₂₋₆-alcényle, phényl-C₃₋₆-alcynyle ou pyridin-C₃₋₆-alcynyle ; R⁶, R⁷ et R⁸ sont chacun indépendamment hydrogène, -NH₂, nitro, furanyle, halogéno, C₁₋₆-alkyle, C₁₋₆-alkyloxy, trifluorométhyle, thiényle, phényle, C₁₋₆-alkylcarbonylamino, aminocarbonyl-C₁₋₆-alkyle ou -C≡C-CH₂-R⁹ ;
où R⁹ est hydrogène, C₁₋₆-alkyle, hydroxy, amino ou C₁₋₆-alkyloxy ; et
hétérocyclyle dans ce qui précède est furanyle, thiényle, pyrrolyle, pyrrolinyle, pyrrolidinyle, dioxolyle, oxazolyle, thiazolyle, imidazolyle, imidazolinyle, imidazolidinyle, pyrazolyle, pyrazolinyle, pyrazolidinyle, isoxazolyle, isothiazolyle, oxadiazolyle, triazolyle, thiadiazolyle, pyranyle, pyridinyle, pipéridinyle, dioxanyle, morpholinyle, dithianyle, thiomorpholinyle, pyridazinyle, pyrimidinyle, pyrazinyle, pipérazinyle, triazinyle, trithianyle, indolizinyle, indolyle, indolinyle, benzofuranyle, benzothiophényle, indazolyle, benzimidazolyle, benzthiazolyle, purinyle, quinolizinyle, quinoléinyle, cinnolinyle, phtalazinyle, quinazolinyle, quinoxalinyle ou naphtyridinyle.

2. Composé selon la revendication 1, **caractérisé en ce que**
chaque X est N ; chaque Y est indépendamment 0 ou CH ; n est 1 ; R¹ est phényle, naphtlalinyle ou hétérocyclyl-C₁₋₆-alkyle où chacun desdits phényle, naphtalinyle ou hétérocyclyl-C₁₋₆-alkyle est éventuellement substitué par un, deux ou trois substituants choisis chacun indépendamment parmi hydrogène, halogéno, C₁₋₆-alkyle, C₁₋₆-alkyloxy, polyhalogéno-C₁₋₆-alkyle, phényle, phényloxy ou cyano, ou deux substituants pris ensemble peuvent former le radical bivalent -O-(CH₂)₂-O-(CH₂)₂-O-(CH₂)₂-O-(CH₂)₂-O- ; R² est hydrogène, C₁₋₆-alkyle ou phényle et R³ est hydroxyle.

3. Composé selon les revendications 1 et 2,
**caractérisé en ce que**
chaque X est N ; chaque Y est CH ; chaque Z est CH ; R¹ est phényle ou hétérocyclyl-C₁₋₆-alkyle où chacun desdits phényle ou hétérocyclyl-C₁₋₆-alkyle est éventuellement substitué par un, deux ou trois substituants choisis chacun indépendamment parmi hydrogène, C₁₋₆-alkyle, phényle ou phényloxy ; R² est hydrogène et R³ est hydroxy.

4. Composé selon les revendications 1, 2 et 3, **caractérisé en ce que** ledit composé est le composé n°3, le composé n°2 et le composé n°35
| | |
|---|---|
| 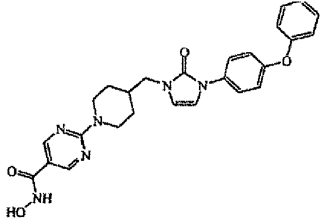 | 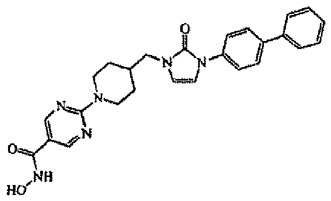 |
| Co. N°2 | Co. N°3 |
| 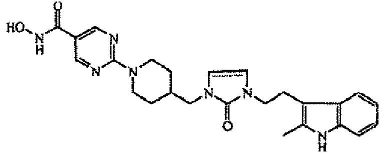 | |
| Co. N°35 | |

5. Composition pharmaceutique comprenant des supports pharmaceutiquement acceptables et à titre de principe actif, une quantité thérapeutiquement efficace d'un composé selon les revendications 1 à 4.

6. Procédé de préparation d'une composition pharmaceutique selon la revendication 5, **caractérisé en ce que** les supports pharmaceutiquement acceptables et un composé selon les revendications 1 à 4 sont mélangés intimement.

7. Composé selon l'une quelconque des revendications 1 à 4, destiné à être utilisé comme médicament.

8. Utilisation d'un composé selon l'une quelconque des revendications 1 à 4, pour la fabrication d'un médicament destiné au traitement de maladies prolifératives.

9. Combinaison d'un agent anticancéreux et d'un inhibiteur d'HDAC selon l'une quelconque des revendications 1 à 4.

10. Procédé de préparation d'un composé selon la revendication 1, **caractérisé par**
a) la réaction d'un intermédiaire de formule (II) avec un acide approprié dans un solvant convenable, donnant un composé de formule (I), dans laquelle R³ est hydroxy, désigné dans la présente composés de formule (I-a), 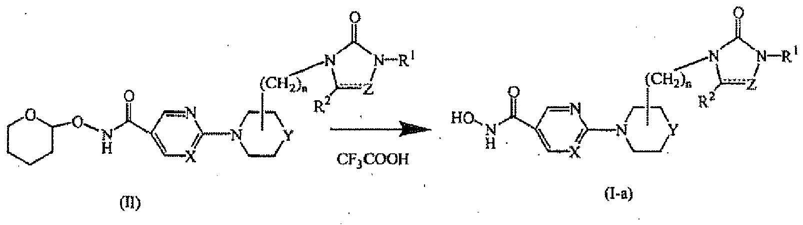
b) la réaction d'un intermédiaire de formule (XV) avec du chlorure d'étain(II) hydraté ou avec de l'hydrogène en présence de palladium sur charbon à 10 %, dans un solvant convenable, avec la formation d'un composé de formule (I), dans laquelle R³ est un radical de formule (a-1) et R⁴ est -NH₂, désigné dans la présente composés de formule (I-b), 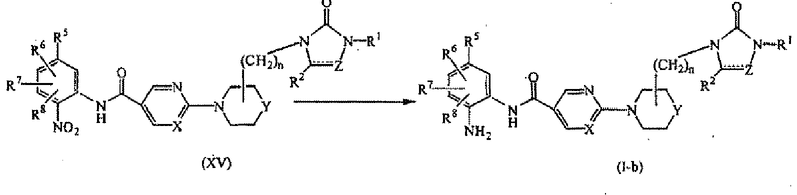
c) la réaction d'un intermédiaire de formule (XVI) avec un acide approprié dans un solvant convenable, avec la formation d'un composé de formule (I-b), 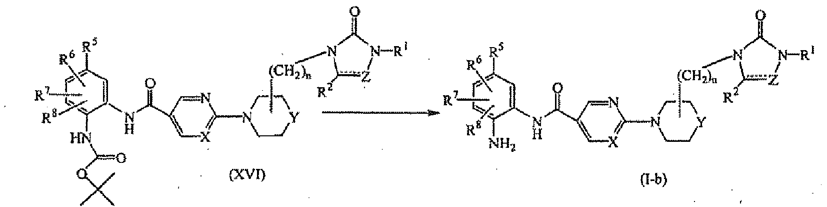
d) la réaction d'un intermédiaire de formule (XVII), dans laquelle TBDMS signifie tert-butyl(diméthyl)silanyle, avec du fluorure de tétrabutylammonium dans un solvant convenable, avec la formation d'un composé de formule (I), dans laquelle R³ est un radical de formule (a-1) et R⁴ est hydroxy, désigné dans la présente composés de formule (I-c).
